(19) Europäisches Patentamt
European Patent Office
Office européen des brevets

(11) Veröffentlichungsnummer: **0 294 599 A2**

(12) # EUROPÄISCHE PATENTANMELDUNG

(21) Anmeldenummer: **88107444.7**

(22) Anmeldetag: **09.05.88**

(51) Int. Cl.⁴: **C07D 471/04 , C07D 495/14 , A61K 31/50 , //(C07D471/04, 237:00,221:00),(C07D495/14, 333:00,237:00,221:00)**

Patentansprüche für folgende Vertragsstaaten: GR + ES.

(30) Priorität: **12.06.87 CH 2206/87**
**08.04.88 CH 1297/88**

(43) Veröffentlichungstag der Anmeldung: **14.12.88 Patentblatt 88/50**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Widmer, Ulrich, Dr.**
**13 Alleeweg**
**CH-4310 Rheinfelden(CH)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

(54) **Tricyclische Pyridonderivate.**

(57) Die neuen Verbindungen der allgemeinen Formel

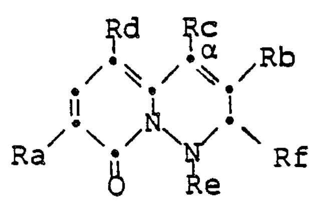

A

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfals durch Halogen, Trifluormethyl, niederes Alkyl, niederes Alkoxy, Nitro, Amino oder mono- oder di(niederes Alkyl)amino substituierte Gruppe der Formel $>C_\alpha$-S-CH=CH- (a), $>C_\alpha$-CH=CH-S- (b) oder $>C_\alpha$-CH=CH-CH=CH- (c), Rd die Gruppe der Formel $-(A^1)_m-(CO)_n-(Q^1A^2)_q-R^1$, m, n und q je die Zahl 0 oder 1, $A^1$ niederes Alkylen, $A^2$ niederes Alkylen, eine direkte Bindung oder die Gruppe -CO-, $Q^1$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine ($C_{3-6}$)-Cycloalkyl-, Hydroxy-, niederes Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes

EP 0 294 599 A2

Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte ($C_{3-7}$)-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe >$N-R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl, Re Wasserstoff, niederes Alkyl, niederes Alkanoyl, Arylcarbonyl, Aryl-niederes Alkanoyl, Aryl-niederes Alkyl oder niederes Alkenyl und Rf Wasserstoff oder niederes Alkyl bedeuten, mit der Massgabe, dass n die Zahl 0 bedeutet, wenn q die Zahl 1 und $A^2$ die Gruppe -CO- bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe -CO- bedeuten, und dass $R^1$ eine von Hydroxy, Cyano, Nitro, Halogen, niederes Alkoxycarbonyl, niederes Alkoxy und $-NR^3R^4$ verschiedene Bedeutung hat, wenn q die Zahl 1 und $A^2$ eine direkte Bindung bedeuten,
und die pharmazeutisch annehmbaren Säureadditionssalze von Verbindungen der Formel A mit einem oder mehreren basischen Substituenten besitzen wertvolle pharmakologische Eigenschaften. Sie sind insbesondere muskelrelaxierend, sedativ-hypnotisch, anxiolytisch und/oder antikonvulsiv wirksam und können bei der Bekämpfung oder Verhütung von Muskelspannungen, Spannungszuständen , Schlaflosigkeit, Angstzuständen und/oder Konvulsionen verwendet werden.

## Tricyclische Pyridonderivate

Die vorliegende Erfindung betrifft Verbindungen der allgemeinen Formel

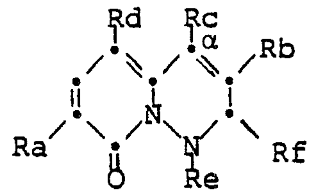

A

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha$-S-CH=CH- (a), $>C_\alpha$-CH-CH-S= (b) oder $>C_\alpha$-CH=CH-CH=CH- (c), Rd die Gruppe der Formel $-(A^1)_m-(CO)_n-(Q^1A^2)_q-R^1$, m, n und q je die Zahl 0 oder 1, $A^1$ niederes Alkylen, $A^2$ niederes Alkylen, eine direkte Bindung oder die Gruppe -CO-, $Q^1$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N-R^5$ enthalten kann, und $R^5$ Wasserstoff, niederes Alkyl, niederes Hydroxyalkyl, niederes Alkoxyalkyl, niederes Alkanoyl oxyalkyl, niederes Alkanoyl, niederes Alkoxycarbonyl, Carbamoyl oder mono- oder di(niederes Alkyl)carbamoyl, Re Wasserstoff, niederes Alkyl, niederes Alkanoyl, Arylcarbonyl, Aryl-niederes Alkanoyl, Aryl-niederes Alkyl oder niederes Alkenyl and Rf Wasserstoff oder niederes Alkyl bedeuten, mit der Massgabe, dass n die Zahl 0 bedeutet, wenn q die Zahl 1 und $A^2$ die Gruppe -CO- bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe -CO- bedeuten, und dass $R^1$ eine von Hydroxy, Cyano, Nitro, Halogen, niederes Alkoxycarbonyl, niederes Alkoxy und $-NR^3R^4$ verschiedene Bedeutung hat, wenn q die Zahl 1 und $A^2$ eine direkte Bindung bedeuten,

und pharmazeutisch annehmbare Säureadditionssalze von Verbindungen der Formel A mit einem oder mehreren basischen Substituenten.

Diese tricyclischen Pyridonderivate besitzen wertvolle pharmakologische Eigenschaften und können zur Bekämpfung oder Verhütung von Krankheiten verwendet werden. Sie sind insbesondere muskelrelaxierend, sedativ-hypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder Konvulsionen verwendet werden.

Gegenstand der vorliegenden Erfindung sind: Die obigen Verbindungen der Formel A und die erwähnten Salze davon als solche; ein Verfahren zu deren Herstellung; die obigen Verbindungen der Formel A und die erwähnten Salze davon zur Anwendung als therapeutische Wirkstoffe; Arzneimittel auf der Basis dieser neuen Wirkstoffe und deren Herstellung; die Verwendung dieser Wirkstoffe bei der Bekämpfung oder

Verhütung von Krankheiten; sowie deren Verwendung zur Herstellung von muskelrelaxierend, sedativ-hypnotisch, anxiolytisch und/oder antikonvulsiv wirksamen Mitteln.

Der Ausdruck "nieder" bezeichnet Reste und Verbindungen mit höchstens sieben, vorzugsweise höchstens vier Kohlenstoffatomen. Der Ausdruck "Alkyl" für sich allein genommen oder in Kombinationen, wie Alkanoyl, Alkanoyloxy und Alkoxyalkyl, bezeichnet geradkettige oder verzweigte, gesättigte Kohlenwasserstoffreste, wie Methyl, Aethyl, Propyl, Isopropyl und t-Butyl. Der Ausdruck "Cycloalkyl" bezeichnet cyclische, gesättigte Kohlenwasserstoffreste, wie Cyclopropyl und Cyclohexyl. Der Ausdruck "Alkoxy" bezeichnet über ein Sauerstoffatom gebundene Alkylgruppen, wie Methoxy und Aethoxy. Der Ausdruck "Hydroxyalkyl" bezeichnet durch Hydroxy substituierte Alkylgruppen, wie Hydroxymethyl und 2-Hydroxyäthyl. Die Ausdrücke "Alkanoyl" und "Alkanoyloxy" bezeichnen Fettsäurereste, wie Acetyl und Acetoxy. Der Ausdruck "Alkylen" bezeichnet geradkettige oder verzweigte, gesättigte Kohlenwasserstoffreste mit zwei freien Valenzen, wie Methylen, 1,2-Aethylen und 1,3-Propylen. Der Ausdruck "Halogen" bezeichnet die vier Formen Fluor, Chlor, Brom und Jod.

Der Ausdruck "Aryl" bezeichnet vorzugsweise Phenylgruppen, welche gegebenenfalls durch Halogen, Trifluormethyl, niederes Alkyl, niederes Alkoxy, Nitro, Amino oder mono- oder di(niederes Alkyl)amino substituiert sind.

Die über ein Kohlenstoffatom gebundenen, 5-gliedrigen gesättigten, partiell ungesättigten oder aromatischen Heterocyclen enthalten als Heteroringglieder vorzugsweise ein Sauerstoff- oder Schwefelatom oder eine Imino- oder niedere Alkylimonogruppe und gegebenenfalls ein oder zwei Stickstoffatome, wobei das Kohlenstoffatom, über das der Heterocyclus gebunden ist, sich vorzugsweise neben einem oder zwischen zwei Heteroatomen befindet. Beispiele solcher Heterocyclen, welche noch wie erwähnt substituiert sein können, sind: 2-Oxazolin-2-yl, 3-Methyl-1,2,4-oxadiazol-5 -yl, 3-Cyclopropyl-1,2,4-oxadiazol-5-yl, 2-Thiazolin-2-yl,2-Tetrahydrofuryl und 2-Thiazolyl.

Der Ausdruck "3- bis 7-gliedriger, gesättigter N-Heterocyclus, der als Ringglied noch ein Sauerstoff- oder Schwefelatom oder die Gruppe $>N-R^5$ enthalten kann" als Bedeutungsmöglichkeit für $-NR^3R^4$ bezeichnet einerseits Heterocyclen mit nur einem Heteroatom, nämlich dem Stickstoffatom, über das sie gebunden sind, und andererseits Heterocyclen mit zwei Heteroatomen, nämlich dem besagten Stickstoffatom und einem Sauerstoff- oder Schwefelatom oder einem zweiten Stickstoffatom.

Beispiele derartiger Heterocyclen, welche noch wie erwähnt substituiert sein können, sind: 2-(niederes Alkoxyalkyl)-1-azetidinyl, 3-(niederes Alkoxy)-1-azetidinyl, 3-Hydroxy-1-azetidinyl, 2-(niederes Hydroxyalkyl)-1-azetidinyl, 2-(niederes Alkanoyloxyalkyl)-1-pyrrolidinyl, 3-Oxo-1-pyrrolidinyl, 2-(niederes Alkoxycarbonyl)-1-pyrrolidinyl, 3-(niederes Alkoxy)-1-pyrrolidinyl, 3-Hydroxy-1-pyrrolidinyl, 2-(niederes Alkoxyalkyl)-1-pyrrolidinyl, 2-(niederes Hydroxyalkyl)-1-pyrrolidinyl, 2-(niederes Hydroxyalkyl)-4-hydroxy-1-pyrrolidinyl, 2-(niederes Alkoxyalkyl)-4-niederes Alkoxy)-1-pyrrolidinyl, 4-Morpholinyl, 2,6-Di(niederes Alkyl)-4-morpholinyl, 4-Thiomorpholinyl, 1-Piperazinyl, 1-(niederes Alkyl)-4-piperazinyl, 1-(niederes Alkoxylalkyl)-4-piperazinyl, 1-(niederes Alkanoyl)-4-piperazinyl, 4-(niederes Hydroxyalkyl)-1-piperidinyl, 4-Oxo-1-piperidinyl, 4-(niederes Alkoxy)-1-piperidinyl, 4-(niederes Alkoxycarbonyl)-1-piperidinyl, 4-Hydroxy-1-piperidinyl, 4-(niederes Alkylcarbamoyl)-1-piperidinyl, 4-(niederes Alkanoyloxy)-1-piperidinyl, 2-(niederes Alkoxyalkyl)-1-piperidinyl, 2-(niederes Hydroxyalkyl)-1-piperidinyl, 3-(niederes Alkoxy)-1-piperidinyl, 4,4-(niederes Alkylendioxy)-1-piperidinyl und 3-Hydroxy-1-piperidinyl.

Das Symbol Ra bedeutet vorzugsweise eine gegebenenfalls durch m-Halogen oder m-Trifluormethyl substituierte Phenylgruppe, wobei die Bedeutungsmöglichkeit Phenyl besonders bevorzugt ist.

Die Symbole Rb und Rc bedeuten zusammen mit dem mit $\alpha$ bezeichneten Kohlenstoffatom vorzugsweise eine gegebenenfalls durch Halogen substituierte Gruppe der Formel $>C_\alpha-S-CH=CH-$ (a) oder $>C_\alpha-CH=CH-CH=CH-$ (c), insbesondere die Gruppe der Formel $>C_\alpha-S-CH=CH-$ oder $>C_\alpha-CH=CCl-CH=CH-$.

In einer bevorzugten Ausführungsform bedeuten Rd die Gruppe $-CONR^3R^4$, $R^3$ niederes Alkyl oder niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen oder durch einen Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann.

In einer besonders bevorzugten Ausführungsform bedeuten dabei $R^3$ niederes Alkyl oder niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen oder durch eine Hydroxy-, niedere Alkoxy-, niedere Hydroxyalkyl- oder niedere Alkoxyalkylgruppe substituierten 1-Azetidinyl-, 1-Pyrrolidinyl-, 1-Piperidinyl- oder 4-Morpholinylgruppe.

In einer ganz besonders bevorzugten Ausführungsform bedeuten dabei $R^3$ niederes Alkyl oder 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl oder 4-Morpholinyl. Re bedeutet vorzugsweise Wasserstoff oder niederes Alkyl, insbesondere Wasserstoff oder Methyl. Rf bedeutet vorzugsweise Wasserstoff oder Methyl, insbesondere Wasserstoff.

Die nachfolgend aufgeführten Verbindungen sind besonders bevorzugte Vertreter der durch die Formel A definierten Stoffklasse;

4-[(10-Chlor-6,7-dihydro-4-oxo-3-phenyl-4H-pyrido[2,1-a]phthalazin-1-yl)carbonyl]morpholin und

(R)-1-[(10-Chlor-6,7-dihydro-4-oxo-3-phenyl-4H-pyrido[2,1-a]phthalazin-1-yl)carbonyl]-2-(methoxymethyl)-pyrrolidin.

Weitere bevorzugte Vertreter der erfindungsgemässen Stoffklasse sind:

1-[(10-Chlor-6,7-dihydro-4-oxo-3-phenyl-4H-pyrido[2,1-a]phthalazin-1-yl)carbonyl]-3-methoxyazetidin,

10-Chlor-6,7-dihydro-N,N-dimethyl-4-oxo-3-phenyl-4H-pyrido[2,1-a]phthalazin-1-carboxamid,

(R)-1-[(4,5-Dihydro-7-oxo-8-phenyl-7H-pyrido[1,2-b]thieno[2,3-d]pyridazin-10-yl)carbonyl]-3-methoxypyrrolidin,,

1-[(4,5-Dihydro-4-methyl-7-oxo-8-phenyl-7H-pyrido[1,2-b]thieno[2,3-d]pyridazin-10-yl)carbonyl]-3-methoxyazetidin,

1-[(4,5-Dihydro-5-methyl-7-oxo-8-phenyl-7H-pyrido[1,2-b]thieno[2,3-d]pyridazin-10-yl)carbonyl]-3-methoxyazetidin und

4,5-Dihydro-N,N-dimethyl-7-oxo-8-phenyl-7H-pyrido[1,2-b]thieno[2,3-d]pyridazin-10-carboxamid.

Die neuen Verbindungen der Formel A und die pharmazeutisch annehmbaren Säureadditionssalze derselben mit einem basischen Substituenten können erfindungsgemäss hergestellt werden, indem man

i) eine Verbindung der allgemeinen Formel

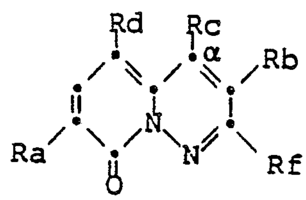 I

worin Ra, Rb, Rc, Rd und Rf obige Bedeutung besitzen, gegebenenfalls in Gegenwart von Ameisensäure mit einem Alkalimetallborhydrid oder einem Derivat davon reduziert, oder

ii) eine Verbindung der allgemeinen Formel

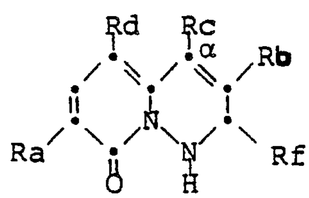 Aa

worin Ra, Rb, Rc, Rd und Rf obige Bedeutung besitzen, mit einer Verbindung der allgemeinen Formel

Re′-X B

worin X eine Abgangsgruppe und Re′ niederes Alkyl, niederes Alkanoyl, Arylcarbonyl, Aryl-niederes Alkanoyl, Aryl-niederes Alkyl oder niederes Alkenyl bedeuten, alkyliert bzw. acyliert, oder

iii) eine Verbindung der allgemeinen Formel

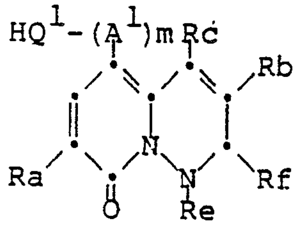 Ab

worin $A^1$, $Q^1$, m, Ra, Rb, Rc, Re und Rf die obige Bedeutung besitzen,

in Gegenwart einer gegebenenfalls starken Base mit einem reaktiven Derivat einer Carbonsäure der allgemeinen Formel

$R^1$-COOH XIII

worin $R^1$ obige Bedeutung besitzt, umsetzt, und

iv) erwünschtenfalls eine erhaltene Verbindung der Formel A mit einem basischen Substituenten in ein pharmazeutisch annehmbares Säureadditionssalz überführt.

Die obigen Verfahren können nach an sich bekannten und jedem Fachmann geläufigen Methoden durchgeführt werden.

Gemäss Verfahrensvariante i) können Verbindungen der Formel A hergestellt werden, worin Re Wasserstoff oder, falls man in Gegenwart von Ameisensäure arbeitet, Methyl bedeutet. Als Reduktionsmittel verwendet man vorzugsweise Natriumcyanoborhydrid unter sauren Bedingungen oder Lithiumborhydrid. Geeignete Lösungsmittel für die Reduktion mit Natriumcyanoborhydrid sind beispielsweise alkoholische Salzsäure, wie methanolische Salzsäure, Essigsäure und Ameisensäure und Mischungen davon mit niederen Alkoholen, wie Methanol, und Aethern, wie Tetrahydrofuran. Für die Reduktion mit Lithiumborhydrid verwendet man als Lösungsmittel vorzugsweise Aether, wie Tetrahydrofuran. Die Reaktion wir vorzugsweise bei Raumtemperatur durchgeführt.

Gemäss Verfahrensvariante ii) können Verbindungen der Formel A hergestellt werden, worin Re niederes Alkyl, niederes Alkanoyl, Arylcarbonyl, Aryl-niederes Alkanoyl, Aryl-niederes Alkyl oder niederes Alkenyl bedeuter. Im Falle von Alkylierungen ist die Abgangsgruppe X vorzugsweise ein Halogenatom, z.B. ein Chlor-, Brom- oder Jodatom, und im Falle von Acylierungen ein Halogenatom, z.B. ein Chloratom, oder die Gruppe Re′-COO-. Geeignete Lösungsmittel sind: halogenierte niedere Kohlenwasserstoffe, wie Methylenchlorid und Chloroform, offenkettige oder cyclische Aether, wie Diäthyläther und Tetrahydrofuran, niedere Fettsäureester, wie Essigester, niedere Alkohole, wie Methanol, Aceton, Dimethylformamid und Dimethylsulfoxid. Im Falle von Acylierungen mit flüssigen Anhydriden kann auch das Anhydrid als Lösungsmittel fungieren. Die Reaktion wird vorzugsweise in einem Bereich von Raumtemperatur bis zur Siedetemperatur des Reaktionsgemisches durchgeführt.

Gemäss Verfahrensvariante iii) können Verbindungen der formel A hergestellt werden, worin Rd eine Gruppe der Formel $-(A^1)_m-Q^1-CO-R^1$ bedeutet, und $A^1$, $Q^1$, $R^1$ und m obige Bedeutung besitzen.

Die Umsetzung einer Verbindung der Formel Ab mit einem reaktiven Derivat einer Carbonsäure der Formel XIII, beispielsweise einem Carbonsäurechlorid, wir zweckmässigerweise in einem inerten organischen Lösungsmittel in Gegenwart einer Base durchgeführt. Falls Re Wasserstoff und $Q^1$ ein Sauerstoffatom bedeuten, verwendet man dabei zweckmässigerweise eine starke Base, wie Natriumhydrid.

Gemäss Verfahrensvariante iv) können Verbindungen der Formel A, welche einen oder mehrere basische Substituenten besitzen, in pharmazeutisch annehmbare Säureadditionssalze übergeführt werden. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succinate, Methansulfonate, p-Toluolsulfonate und dergleichen.

Die als Ausgangsstoffe verwendeten Verbindungen der Formel I können hergestellt werden, indem man

a) eine Verbindung der allgemeinen Formel

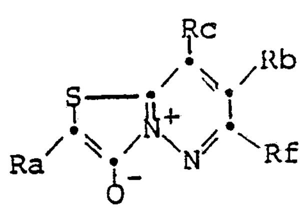

II

worin Ra, Rb, Rc und Rf obige bedeutung besitzen, bei erhöhter Temperatur mit einer Verbindung der allgemeinen Formel

HC≡C-Rd′ III

oder

$H_2C=CH$-Rd′ IV

worin Rd′ Cyano, Nitro oder die Gruppe der Formel $-CO-(Q^1A^2)_q-R^1$ bedeutet, und q, $A^2$, $Q^1$ und $R^1$ die obige Bedeutung besitzen,

oder mit Phenylvinylsulfoxid umsetzt und das erhaltene Cycloadditionsprodukt gegebenenfalls mit einer starken Base behandelt, oder

b) eine Verbindung der allgemeinen Formel

ROOC-C(Ra) = CHR′ V

worin R niederes Alkyl und R′ Wasserstoff oder niederes

Alkoxy bedeuten, und Ra die obige Bedeutung besitzt, wenn R′ Wasserstoff bedeutet, bei erhöhter Temperatur oder, wenn R′ niederes Alkoxy bedeutet, in Gegenwart einer starken Base mit einer Verbindung der allgemeinen Formel

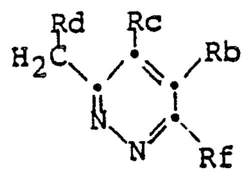

VI

worin Rb, Rc, Rd und Rf obige Bedeutung besitzen, umsetzt und das erhaltene Cyclokondensationsprodukt, wenn R′ Wasserstoff bedeutet, dehydriert, oder

c) eine Verbindung der Formel I, welche eine veresterte Carboxygruppe besitzt, hydrolysiert, oder

d) eine Carbonsäure der allgemeinen Formel

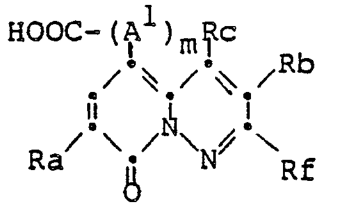

Ia

worin $A^1$, Ra, Rb, Rc, m und Rf obige Bedeutung besitzen,
mit einem Alkohol der allgemeinen Formel

$HO-A^{21}-R^1$ VII

worin $A^{21}$ niederes Alkylen oder eine direkte Bindung bedeutet, und $R^1$ die obige Bedeutung besitzt, verestert, oder

e) eine Carbonsäure der obigen Formel Ia oder eine Carbonsäure der allgemeinen Formel

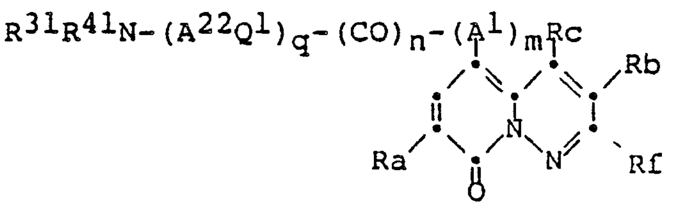

VIIIa

worin $A^{22}$ niederes Alkylen oder die Gruppe -CO- und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- oder Schwefelatom oder die Gruppe $>N-R^5$ enthalten kann, und $A^1$, $Q^1$, Ra, Rb, Rc, $R^5$, m, n, q und Rf obige Bedeutung besitzen,
oder ein reaktives Derivat davon mit einem Amin der allge meinen Formel

$HNR^2-A^{21}-R^1$ IX

oder

$HNR^3R^4$ X

worin $A^{21}$, $R^1$, $R^2$, $R^3$ und $R^4$ die obige Bedeutung besitzen,
bzw. mit Ammoniak oder einem mono- oder di(niederen Alkyl)amin in das entsprechende Amid überführt, oder

f) eine Verbindung der allgemeinen Formel

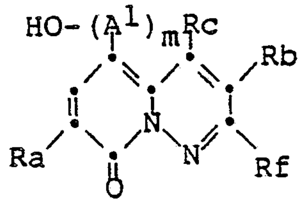

Ib'

worin $A^1$, Ra, Rb, Rc, m und Rf obige Bedeutung besitzen,
in Gegenwart einer Base mit einer Verbindung der allgemeinen Formel

$X\text{-}A^{21}\text{-}R^1$   XI

worin X eine Abgangsgruppe bedeutet, und $A^{21}$ und $R^1$ die obige Bedeutung besitzen,
bzw. eine Verbindung der Formel I, welche eine freie Hydroxygruppe besitzt, mit einer Verbindung der allgemeinen Formel

R-X   XII

worin R niederes Alkyl bedeutet, und X obige Bedeutung besitzt,
umsetzt, oder

g) eine Verbindung der allgemeinen Formel

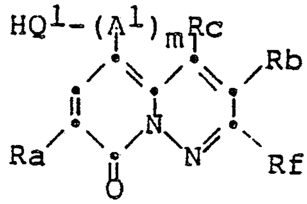

Ib

worin $A^1$, $Q^1$, Ra, Rb, Rc, m und Rf obige Bedeutung besitzen,
in Gegenwart eines säurebindenden Mittels mit einem reaktiven Derivat einer Carbonsäure der allgemeinen Formel

$R^1\text{-}COOH$   XIII

worin $R^1$ die obige Bedeutung besitzt, umsetzt, oder

h) eine Verbindung der allgemeinen Formel

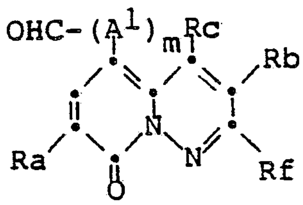

Ic

worin $A^1$, Ra, Rb, Rc, m und Rf obige Bedeutung besitzen,
in Gegenwart eines Reduktionsmittels mit einem Amin der obigen Formel IX oder X umsetzt, oder

i) eine Verbindung der allgemeinen Formel

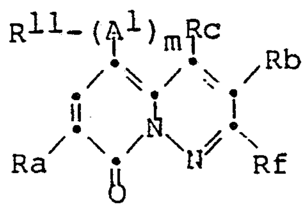

Id

worin $R^{11}$ Nitro, Cyano oder niederes Alkoxycarbonyl bedeutet, und $A^1$, Ra, Rb, Rc, m und Rf obige Bedeutung besitzen,
oder eine Verbindung der obigen Formel Ia oder ein reaktives Derivat davon reduziert, oder

j) einen Alkohol der obigen Formel Ib′ oder einen Alkohol der allgemeinen Formel

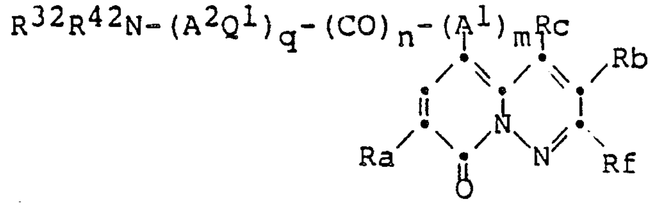

Ie

worin $A^1$, $A^2$, $Q^1$, Ra, Rb, Rc, m, n, q und Rf obige Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigen N-Heterocyclus bedeuten, der als Ringglied noch ein Sauerstoff- oder Schwefelatom oder die Gruppe >N-$R^5$ enthalten kann, und $R^5$ die obige Bedeutung besitzt,
oxidiert, oder

k) ein Isocyanat der allgemeinen Formel

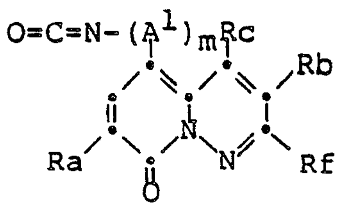

VIIIb

oder O=C-N-$R^{33}$ XIV

worin $A^1$, Ra, Rb, Rc, m und Rf obige Bedeutung besitzen, und $R^{33}$ Wasserstoff, niederes Alkyl oder $(C_{3-7})$-Cycloalkyl bedeutet,
mit einem niederen Alkohol oder einem Amin der obigen Formel X bzw. mit einer Verbindung der obigen Formel Ib umsetzt, oder

l) eine Verbindung der allgemeinen Formel

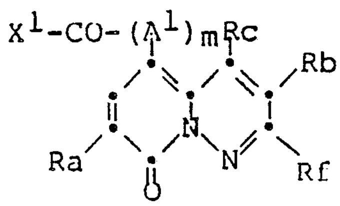

VIIIc

worin $X^1$ ein Halogenatom bedeutet, und $A^1$, Ra, Rb, Rc, m und Rf obige Bedeutung besitzen,
mit einem niederen Alkylmagnesiumhalogenid umsetzt, oder

m) eine Verbindung der allgemeinen Formel

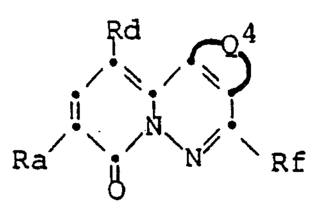

Ih

worin $Q^4$ die obige Gruppe (a) oder (b) bedeutet und Ra, Rd und Rf obige Bedeutung besitzen,
am Thiophenring halogeniert, oder

n) eine Verbindung der obigen Formel VIIIc in Gegenwart einer Base mit einer Verbindung der allgemeinen Formel
HYN=C($NH_2$)-R″ XV,

$H_2N\text{-}CHR''\text{-}CHR'''\text{-}Y'H$ XVI
oder
$H_2N\text{-}NH\text{-}C(R'')=Y''$ XVII

worin Y ein Sauerstoffatom oder die Gruppe -NR'''-, Y' ein Sauerstoffatom oder die Gruppe -NH-, Y'' ein Sauerstoff- oder Schwefelatom und R'' und R''' je Wasserstoff oder niederes Alkyl bedeuten,
umsetzt und das erhaltene Produkt cyclisiert, oder

o) eine Verbindung der allgemeinen Formel

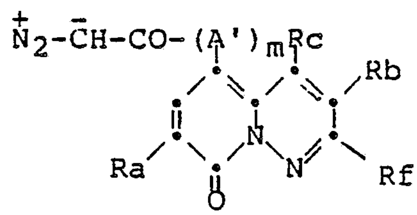
VIIId

worin A' $C_{1-6}$-Alkylen bedeutet, und Ra, Rb, Rc, Rf und m obige Bedeutung besitzen,
mit einem niederen Alkohol umsetzt, oder

p) eine Carbonsäure der Formel Ia, worin m die Zahl 0 bedeutet, decarboxyliert, oder

q) eine Verbindung der Formel I, worin Rd Wasserstoff bedeutet, am Pyridonring halogeniert, oder

r) in einer Verbindung der allgemeinen Formel

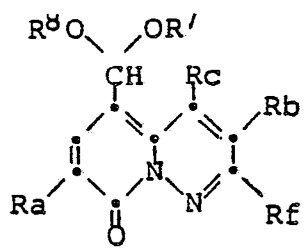
VIIIe

worin $R^7$ und $R^8$ je niederes Alkyl oder zusammen niederes Alkylen bedeuten, und Ra, Rb, Rc und Rf obige Bedeutung besitzen,
die Acetalgruppe spaltet, oder

s) eine Verbindung der allgemeinen Formel

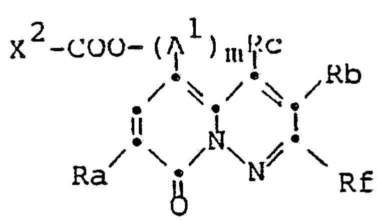
VIIIf

worin $X^2$ Phenoxy bedeutet, und $A^1$, Ra, Rb, Rc, Rf und m obige Bedeutung besitzen,
mit einem Amin der obigen Formel X umsetzt.

Bei verschiedenen der obigen Verfahren müssen die in den Ausgangsstoffen allfällig vorhandenen reaktionsfähigen Amino-, Carboxy- und/oder Hydroxylgruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten.

Gemäss Verfahrensvariante a) können Verbindungen der Formel I hergestellt werden, worin Rd Wasserstoff, Cyano, Nitro oder die Gruppe der Formel $\text{-CO-}(Q^1A^2)_q\text{-}R^1$ bedeutet und q, $A^2$, $Q^1$ und $R^1$ obige Bedeutung besitzen. Die Umsetzung erfolgt zweckmässigerweise in einem inerten Lösungsmittel, das bei erhöhter Temperatur, vorzugsweise oberhalb 80 °C siedet. Geeignete Lösungsmittel sind beispielsweise aromatische Kohlenwasserstoffe, wie Benzol, Toluol und Xylol, wobei die Reaktion vorzugsweise bei der Rückflusstemperatur des Lösungsmittels durchgeführt wird.

Bei der Umsetzung einer Verbindung der Formel II mit einer Verbindung der Formel III oder mit Phenylvinylsulfoxid erhält man, wenn man bei erhöhter Temperatur arbeitet, direkt die entsprechende

Verbindung der Formel I. Bei der Umsetzung einer Verbindung der Formel II mit einer Verbindung der Formel IV erhält man als Cycloadditionsprodukt zunächst die entsprechende Epithio-Verbindung der allgemeinen Formel

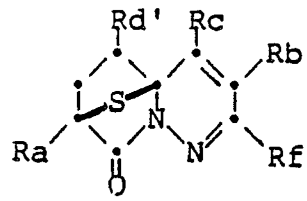

XVIII

worin Ra, Rb, Rc, Rd′ und Rf obige Bedeutung besitzen, welche anschliessend durch Behandeln mit einer starken Base in die entsprechende Verbindung der Formel I übergeführt wird. Geeignete Basen sind beispielsweise die niederen Alkalimetallalkoholate, wie Natriummethylat, wobei man als Lösungsmittel zweckmässigerweise den entsprechenden niederen Alkohol verwendet. Die Reaktion wird vorzugsweise bei der Rückflusstemperatur des Lösungsmittels durchgeführt.

Die Reaktion einer Verbindung der Formel V, worin R′ Wasserstoff bedeutet, mit einer Verbindung der Formel VI gemäss Verfahrensvariante b) kann ohne Lösungsmittel oder in Gegenwart eines bei erhöhter Temperatur seidenden Lösungsmittels durchgeführt werden. Geeignete Lösungsmittel sind beispielsweise aromatische Kohlenwasserstoffe, wie Benzol, Toluol und Xylol. Die Cyclokondensation wird jedoch vorzugsweise ohne Lösungsmittel in einem Temperaturbereich von etwa 80 °C bis etwa 150 °C durchgeführt. Das so erhaltene Cyclokondensationsprodukt, nämlich eine Verbindung der allgemeinen Formel

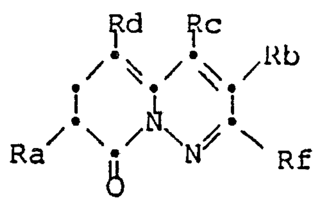

XIX

worin Ra, Rb, Rc, Rd und Rf obige Bedeutung besitzen, wird anschliessend mit einem geeigneten Oxidationsmittel, wie Mangandioxid, dehydriert. Geeignete Lösungsmittel sind beispielsweise aromatische Kohlenwasserstoffe, wie Benzol, Toluol und Xylol. Die Dehydrierung wird vorzugsweise in einem Temperaturbereich von etwa Raumtemperatur bis zur Siedetemperatur des gewählten Lösungsmittels, vorzugsweise bei der Siedetemperatur, durchgeführt.

Durch Reaktion einer Verbindung der Formel V, worin R′ niederes Alkoxy bedeutet, in Gegenwart einer starken Base, wie Natriumhydrid, und in einem inerten Lösungsmittel, vorzugsweise in einem Aether, wie Tetrahydrofuran, mit einer Verbindung der Formel VI gemäss Verfahrensvariante b) erhält man die entsprechende Verbindung der Formel I. Die Reaktionstemperatur liegt in einem Bereich von Raumtemperatur bis zur Seidetemperatur der Reaktionsmischung.

Verbindungen der Formel I, welche eine veresterte Carboxygruppe besitzen, können gemäss Verfahrensvariante c) hydrolysiert werden, wobei man die entsprechenden freien Carbonsäuren erhält. Die Hydrolyse kann nach an sich bekannten Methoden durchgeführt werden. Die Hydrolyse wird vorzugsweise mit einem Alkalimetallhydroxid, wie Natriumhydroxid und Kaliumhydroxid, in einem niederen Alkohol, wie Me thanol und Aethanol, oder in einem Gemisch aus einem niede ren Alkohol und Wasser durchgeführt. Die Reaktionstemperatur liegt zweckmässigerweise in einem Bereich von Raumtemperatur bis zur Siedetemperatur des Reaktionsgemisches, wobei man vorzugsweise bei der Siedetemperatur des Reaktionsgemisches arbeitet.

Durch Veresterung einer Carbonsäure der Formel Ia mit einem Alkohol der Formel VII gemäss Verfahrensvariante d) können Verbindungen der Formel I hergestellt werden, worin Rd die Gruppe der Formel $-(A^1)_m-CO-O-A^{21}-R^1$ bedeutet und $A^1$, $A^{21}$, $R^1$ und m obige Bedeutung besitzen. Die Veresterung kann beispielsweise in Gegenwart eines Veresterungsreagenzes in einem inerten organischen Lösungsmittel durchgeführt werden. Geeignete Reagenzien sind beispielsweise N-Methyl-2-chlorpyridiniumjodid und dergleichen, organische Sulfonsäurehalogenide, wie Methylsulfonylchlorid, p-Toluolsulfonylchlorid und Mesitylensulfonsäurechlorid, und dergleichen. Geeignete Lösungsmittel sind beispielsweise halogenierte Kohlenwasserstoffe, wie Methylenchlorid, Chloroform und dergleichen. Geignete Basen sind beispielsweise tertiäre

Amine, wie Triäthylamin, Tri-n-butylamin und dergleichen. Die Reaktion wird vorzugsweise in einem Temperaturbereich von Raumtemperatur bis zur Rückflusstemperatur des Lösungsmittels durchgeführt.

Die gewünschte Veresterung kann auch dadurch bewerkstelligt werden, dass man die Carbonsäure der Formel Ia zunächst in ein reaktives Derivat überführt und dieses dann in Gegenwart einer Base mit einem alkohol der Formel VII umsetzt. Als reaktive Derivate verwendet man vorzugsweise die entsprechenden Carbonsäurechloride. Als Basen eignen sich beispielsweise die bereits erwähnten tertiären Amine. Die Reaktion wird vorzugsweise in einem Temperaturbereich von etwa Raumtemperatur bis zur Rückflusstemperatur des Reaktionsgemisches durchgefürht, wobei man zweckmässigerweise bei Raumtemperatur arbeitet.

Die Veresterung mit einem Alkohol der Formel VII, worin $A^{21}$ niederes Alkylen und $R^1$ Wasserstoff bedeuten, d.h. mit einem niederen Alkohol, kann auch durch Umsetzen der Carbonsäure mit einem di-(niederen Alkyl)acetal des N,N-Dimethylformamids durchgeführt werden. Die Umsetzung mit einem di-(niederen Alkyl)acetal des N,N-Dimethylformamids wird vorzugsweise in einem inerten Lösungsmittel, beispielsweise in einem aromatischen Kohlenwasserstoff, wie Benzol, bei der Rückflusstemperatur des Reaktionsgemisches durchgeführt.

Durch Reaktion einer Carbonsäure der Formel Ia oder eines reaktiven Derivates davon mit einem Amin der Formel IX oder X gemäss Verfahrensvariante e) können Verbindungen der Formel I hergestellt werden, worin Rd de Gruppe $-(A^1)_m-CO-NR^2-A^{21}-R^1$ oder $-(A^1)_m-CO-NR^3R^4$ bedeutet und $A^1$, $A^{21}$, $R^1$, $R^2$, $R^3$, $R^4$ und m obige Bedeutung besitzen.

Durch Umsetzen einer Carbonsäure der Formel VIIIa oder eines reaktiven Derivates davon mit Ammoniak oder einem mono- oder di(niederen Alkyl)amin gemäss Verfahrensvariante e) können entsprechende Verbindungen der Formel I hergestellt werden, worin $R^1$ eine Gruppe der Formel $-NR^3R^4$ und $R^3$ und $R^4$ zusammen mit dem Stickstoffatom einen 3- bis 7-gliedrigen, gegebenenfalls durch eine oder zwei niedere Alkylgruppen und durch eine Carbamoyl- oder mono- oder di(niedere Alkyl)carbamoylgruppe substituierten, gesättigten N-Heterocyclus bedeuten, der als Ringglied noch ein Sauerstoff- oder Schwefelatom oder die Gruppe $>N-R^5$ enthalten kann, und $R^5$ obige Bedeutung besitzt.

Verwendet man als Ausgangsstoff die freie Carbonsäure der Formel Ia oder VIIIa, so wird die Amidierungsreaktion vorzugsweise in Gegenwart eines Kondensationsmittels, wie N-Methyl-2-chlorpyridiniumjodid, in einem inerten organischen Lösungsmittel und in Gegenwart einer Base durchgeführt. Geeignete Lösungsmittel sind beispielsweise aromati sche Kohlenwasserstoffe wie Benzol, Toluol und Xylol. Geeignete Basen sind beispielsweise die weiter oben erwähnten tertiären Amine. Bevorzugte reaktive Carbonsäurederivate, welche in Gegenwart einer Base direkt mit dem entsprechenden Amin umgesetzt werden können, sind die entsprechenden Carbonsäurechloride. Geeignete Basen sind wiederum die vorerwähnten tertiären Amine. Geeignete Lösungsmittel sind beispielsweise aromatische Kohlenwasserstoffe, wie Benzol, Toluol und Xylol, und Aether, wie Dioxan. Die Reaktion wird in beiden Fällen vorzugsweise in einem Bereich von Raumtemperatur bis Rückflusstemperatur des Reaktionsgemisches durchgeführt.

Gemäss Verfahrensvariante f) können einerseits Verbindungen der Formel I hergestellt werden, worin Rd eine Gruppe der Formel $-(A^1)_m-O-A^{21}-R^1$ bedeutet, und $A^1$, $A^{21}$, $R^1$ und m obige Bedeutung besitzen, und andererseits Verbindungen der Formel I, welche eine in Form eines niederen Alkyläthers verätherte Hydroxygruppe besitzen.

Die Umsetzung einer Verbindung der Formel Ib′ mit einer Verbindung der Formel XI bzw. die Umsetzung einer Verbindung der Formel I, welche eine freie Hydroxygruppe besitzt, mit einer Verbindung der Formel XII wird zweckmässigerweise in einem inerten organischen Lösungsmittel, wie N,N-Dimethylformamid oder dergleichen, durchgeführt, wobei man als Base zweckmässigerweise eine starke Base, z.B. ein Alkalimetallhydrid oder -hydroxyd, wie Natriumhydrid, Kaliumhydroxyd und Natriumhydroxyd, verwendet. Man arbeitet zweckmässigerweise in einem Bereich von 0°C bis Raumtemperatur. Die mit X bezeichnete Abgangsgruppe ist vorzugsweise ein Halogenatom, insbesondere ein Chlor-, Brom- oder Jodatom, oder eine Alkyl- oder Arylsulfonyloxygruppe, beispielsweise eine Methansulfonyloxy- oder p-Toluolsulfonyloxygruppe. Im Falle der Herstellung von niederen Alkyläthern kann X auch eine niedere Alkoxysulfonyloxygruppe bedeuten, d.h. dass das Alkylierungsmittel in diesem Fall ein Di(niederes Alkyl)sulfat, wie Dimethylsulfat, ist.

Gemäss Verfahrensvariante g) können Verbindungen der Formel I hergestellt werden, worin Rd eine Gruppe der Formel $-(A^1)_m-Q^1-CO-R^1$ bedeutet, und $A^1$, $Q^1$, $R^1$ und m obige Bedeutung besitzen.

Die Umsetzung einer Verbindung der Formel Ib mit einem reaktiven Derivat einer Carbonsäure der Formel XIII, beispielsweise einem Carbonsäurechlorid, wird zweckmässigerweise in einem inerten organischen Lösungsmittel in Gegenwart eines säurebindenden Mittels, beispielsweise einem tertiären Amin, durchfgeführt. Als Lösungsmittel eignen sich beispielsweise aromatische Kohlenwasserstoffe, wie Benzol,

Toluol und Xylol, und halogenierte Kohlenwasserstoffe, wie Methylenchlorid. Wenn $R^1$ niederes Alkyl bedeutet, können auch entsprechende Carbonsäureanhydride verwendet werden, wobei man in diesem Fall als Lösungsmittel und als säurebindendes Mittel zweckmässigerweise Pyridin verwendet. Die Reaktion wird vorzugsweise in einem temperaturbereich von etwa 0° C bis Siedetemperatur des Lösungsmittels durchgeführt.

Gemäss Verfahrensvariante h) können Verbindungen der Formel I hergestellt werden, worin Rd eine Gruppe der Formel $-(A^1)_m-CH_2-NR^2A^{21}-R^1$ oder $-(A^1)_m-CH_2-NR^3R^4$ bedeutet und $A^1$, $A^{21}$, $R^1$, $R^2$, $R^3$, $R^4$ und m obige Bedeutung besitzen. Die Reaktion wird vorzugsweise in einem niederen Alkohol als Lösungsmittel und mit Natriumcyanoborhydrid als Reduktionsmittel durchgeführt, wobei man zweckmässigerweise bei Raumtemperatur arbeitet und das Amin in Form seines Hydrochlorides einsetzt.

Gemäss Verfahrensvariante i) können Verbindungen der Formel I hergestellt werden, worin Rd eine Gruppe der Formel $-(A^1)_m-R^{12}$ und $R^{12}$ Amino, Aminomethyl, Hydroxymethyl oder Methyl bedeuten und $A^1$ und m obige Bedeutung besitzen. Die Wahl des geeigneten Reduktionsmittels richtet sich einerseits nach dem verwendeten Ausgangsstoff und andererseits nach dem gewünschten Produkt. Eine Verbindung der Formel Id, worin $R^{11}$ Cyano bedeutet kann beispielsweise mit Diboran in Tetrahydrofuran zur entsprechenden Aminomethylverbindung reduziert werden. Eine Verbindung der Formel Id, worin $R^{11}$ Nitro bedeutet, kann beispielsweise mit Natriumsulfid in einem niederen Alkohol, wie Methanol, zur entsprechenden Aminoverbindung reduziert werden. Eine Verbindung der Formel Id worin $R^{11}$ niederes Alkoxycarbonyl bedeutet, kann mit Lithiumborhydrid, und das Säurechlorid einer Verbindung der Formel Ia mit Natriumborhydrid in Tetrahydrofuran und/oder Dimethylformamid zur entsprechenden Hydroxymethylverbindung reduziert werden. Eine Carbonsäure der Formel Ia kann beispielsweise mit Boran/Tetrahydrofuran-Komplex oder Boran/Methylsulfid-Komplex in Tetrahydrofuran zur entsprechenden Methylverbindung reduziert werden.

Gemäss Verfahrensvariante j) können Verbindungen der Formel I hergestellt werden, worin Rd eine Gruppe der Formel $-(A^1)_m-CHO$ oder $-(A^1)_m-(CO)_n-(Q^1A^2)_q-NR^{34}R^{44}$ und $R^{34}$ und $R^{44}$ zusammen einen 3- bis 7-gliedrigen, gegebenenfalls durch eine oder zwei niedere Alkylgruppen und durch eine Oxogruppe substituierten, gesättigten N-Heterocyclus bedeuten, der als Ringglied noch ein Sauerstoff- oder Schwefelatom oder die Gruppe >N-$R^5$ enthalten kann, und $A^1$, $A^2$, $Q^1$, $R^5$, m, n und q obige Bedeutung besitzen. Die Oxidation von Alkoholen der Formeln Ib′ und Ie kann nach an sich bekannten und jedem Fachmann geläufigen Methoden durchgeführt werden. Man kann die gewünschte Oxidation beispielsweise mit Mangandioxid in einem halogenierten Kohlenwasserstoff, wie Methylenchlorid bei Raumtemperatur bewerkstelligen. Die gewünschte Oxidation kann jedoch auch mit Pyridiniumchlorochromat in einem halogenierten Kohlenwasserstoff, wie Methylenchlorid, bei Raumtemperatur, oder mit Dimethylsulfoxid/Trifluoressigsäureanhydrid in einem halogenierten Kohlenwasserstoff, wie Methylenchlorid, bei Temperaturen von etwa -70° C bewerkstelligt werden.

Gemäss Verfahrensvariante k) können durch Umsetzung eines Isocyanates der Formel VIIIb mit einem niederen Alkohol oder einem Amin der Formel X, bzw. durch Umsetzen eines Isocyanates der Formel XIV mit einer Verbindung der Formel Ib Verbindungen der Formel I hergestellt werden, worin Rd eine Gruppe der Formel $-(A^1)_m-NHCO-R^{13}$, $-(A^1)_m-NHCO-NR^3R^4$ oder $-(A^1)_m-Q^1-CO-NH-R^{33}$ und $R^{13}$ niederes Alkoxy bedeuten, und $A^1$, $Q^1$, $R^3$, $R^{33}$, $R^4$ und m obige Bedeutung besitzen. Diese Umsetzung wird zweckmässigerweise in einem inerten Lösungsmittel, beispielsweise in einem aromatischen Kohlenwasserstoff, wie Benzol, Toluol oder Xylol, in einem halogenierten Kohlenwasserstoff, wie Methylenchlorid, oder in einem Aether, wie Dioxan, durchgeführt. Die Reaktion wird vorzugsweise in einem Temperaturbereich von etwa Raumtemperatur bis zur Siedetemperatur des Reaktionsgemisches durchgeführt. Verwendet man ein Isocyanat der Formel XIV, worin $R^{33}$ Wasserstoff bedeutet, als Ausgangsstoff, so wird dieses zweckmässigerweise in geschützter Form eingesetzt. Eine besonders geeignete Schutzgruppe ist dabei die Trichloracetylgruppe, welche nach erfolgter Umsetzung beispielsweise mit Kaliumcarbonat in Wasser durch Hydrolyse wieder entfernt werden kann.

Gemäss Verfahrensvariante l) können Verbindungen der Formel I hergestellt werden, worin Rd eine Gruppe der Formel $-(A^1)_m-CO-R^{14}$ und $R^{14}$ niederes Alkyl bedeuten, und $A^1$ und m obige Bedeutung besitzen. Als Lösungsmittel verwendet man vorzugsweise Aether, wie Tetrahydrofuran. Die Reaktion wird vorzugsweise in einem Temperaturbereich von -78° C bis Raumtemperatur durchgeführt.

Gemäss Verfahrensvariante m) können Verbindungen der Formel I hergestellt werden, worin Rb und Rc zusammen mit dem mit $\alpha$ bezeichneten Kohlenstoffatom eine durch Halogen substituierte Gruppe der Formel $>C_\alpha$-S-CH=CH- (h) oder $>C_\alpha$-CH=CH-S- (i) bedeuten. Als Halogenierungsmittel verwendet man vorzugsweise elementares Halogen, beispielsweise elementares Brom. Geeignete Lösungsmittel sind beispielsweise halogenierte Kohlenwasserstoffe, wie Chloroform. Man arbeitet zweckmässigerweise in einem Temperaturbereich von 0° C bis etwa Raumtemperatur.

Gemäss Verfahrensvariante n) können Verbindungen der Formel I hergestellt werden, worin Rd eine

Gruppe der Formel $-(A^1)_m-R^{15}$ und $R^{15}$ einen über ein Kohlenstoffatom gebundenen, 5-gliedrigen, gegebenenfalls durch eine oder zwei niedere Alkylgruppen substituierte, partiell ungesättigten oder aromatischen Heterocyclus bedeuten, und $A^1$ und m obige Bedeutung besitzen. Die Umsetzung einer Verbindung der Formel VIIIc mit einer Verbindung der Formel XVI, XVII oder XVIII wird zweckmässigerweise in einem inerten Lösungsmittel, beispielsweise in einem halogenierten Kohlenwasserstoff, wie Methylenchlorid, oder in einem aromatischen Kohlenwasserstoff, wie Benzol, Toluol oder Xylol, und in einem Temperaturbereich von etwa 0 °C bis zur Rückflusstemperatur des Reaktionsgemisches durchgeführt. Geeignete Basen sind beispielsweise die bereits früher erwähnten tertiären Amine. Die Cyclisierung des so erhaltenen Produktes kann nach an sich bekannten und jedem Fachmann geläufigen Methoden durchgeführt werden. Die Cyclisierung kann beispielsweise in Gegenwart katalytischer Mengen einer starken Säure wie p-Toluolsulfonsäure, unter Entfernung des gebildeten Reaktionswassers mittels eines Schleppers, wie Toluol, bewerkstelligt werden. Die Cyclisierung kann aber auch mittels Azodicarbonsäurediäthylester/Triphenylphosphin in einem Aether, wie Tetrahydrofuran, bewerkstelligt werden.

Gemäss Verfahrensvariante o) können Verbindungen der Formel I hergestellt werden, worin Rd eine Gruppe der Formel $-(A')_m-CH_2-R^{16}$, $A'$ $C_{1-6}$-Alkyl und $R^{16}$ niederes Alkoxycarbonyl bedeuten, und m obige Bedeutung besitzt. Die Reaktion eines Diazoketons der Formel VIIId mit einem niederen Alkohol wird vorzugsweise in Gegenwart eines Silberkatalysators, wie Silveroxid, durchgeführt, wobei man als Lösungsmittel vorzugsweise den niederen Alkohol verwendet. Die Reaktion wird bei erhöhter Temperatur, vorzugsweise bei der Siedetemperatur des Reaktionsgemisches durchgeführt.

Gemäss Verfahrensvariante p) können Verbindungen der Formel I hergestellt werden, worin Rd Wasserstoff bedeutet. Die Decarboxylierung einer Carbonsäure der Formel Ia wird vorzugsweise durch trockenes Erhitzen, insbesondere durch trockenes Erhitzen im Vakuum auf Temperaturen von etwa 200° bis etwa 300 °C bewerkstelligt.

Gemäss Verfahrensvariante q) können Verbindungen der Formel I hergestellt werden, worin Rd Halogen bedeutet. Für die vorliegende Halogenierung geeignete Halogenierungsmittel sind N-Halogenimide und -amide, wie N-Chlorsuccinimid, N-Bromsuccinimid, N-Chloracetamid und dergleichen. Als Lösungsmittel verwendet man vorzugsweise einen halogenierten Kohlenwasserstoff, wie Methylenchlorid, Chloroform, Tetrachlorkohlenstoff und dergleichen. Die Reaktion kann in einem Temperaturbereich von etwa 0 °C bis zur Seidetemperatur des Reaktionsgemisches durchgeführt werden. Vorzugsweise arbeitet man bei Raumtemperatur.

Durch Spaltung der Acetalgruppe in einer Verbindung der Formel VIIIe gemäss Verfahrensvariante r) können Verbindungen der Formel I hergestellt werden, worin Rd die Gruppe -CHO bedeutet. Die Spaltung wird vorzugsweise durch Umacetalisieren in Gegenwart einer Säure, wie p-Toluolsulfonsäure, und eines Ketons, wie Cyclohexanon, Aceton und dergleichen bewerkstelligt. Die Reaktion kann in einem Temperaturbereich von Raumtemperatur bis zur Seidetemperatur des Reaktionsgemisches durchgeführt werden.

Gemäss Verfahrensvariante s) können Verbindungen der Formel I hergestellt werden, worin $R^1$ eine Gruppe der Formel $-(A^1)_m-OCO-NR^3R^4$ bedeutet. Für den vorliegenden Zweck geeignete Lösungsmittel sind beispielsweise Aether, wie Tetrahydrofuran, Dioxan und Diäthyläther, N,N-Dimethylformamid und Dimethylsulfoxid. Die Reaktion wird zweckmässigerweise bei Raumtemperatur durchgeführt.

Die als Ausgangsstoffe verwendeten Verbindungen der Formeln II, VIIIa, VIIIb, VIIIc, VIIId, VIIIe und VIIIf können wie nachfolgend beschrieben hergestellt werden.

Die Verbindungen der Formel II können beispielsweise dadurch hergestellt werden, dass man eine Verbindung der allgemeinen Formel

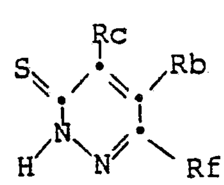 XXI

worin Rb, Rc und Rf obige Bedeutung besitzen, mit einer Verbindung der allgemeinen Formel

$Ra-CHX^2-COX^1$ XXII oder 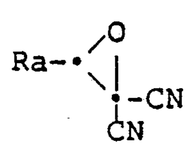 XXIII

worin $X^1$ und $X^2$ je Halogen bedeuten und Ra obige Bedeutung besitzt,
umsetzt. Die Reaktion einer Verbindung der Formel XXI mit einer Verbindung der Formel XXII, worin $X^1$ vorzugsweise Chlor und $X^2$ vorzugsweise Brom bedeuten, wird vorzugsweise bei Raumtemperatur in einem halogenierten Kohlenwasserstoff, wie Chloroform, durchgeführt, worauf man mit einem basischen Amin, wie Triäthylamin, behandelt. Die Reaktion einer Verbindung der Formel XXI mit einer Verbindung der Formel XXIII wird vorzugsweise in einem inerten Lösungsmittel, wie Aceton, N,N-Dimethylformamid, Dimethylsulfoxid und dergleichen, bei Raumtemperatur durchgeführt.

Die Carbonsäuren der Formel VIIIa können durch Hydrolyse der entsprechenden niederen Alkylester der Formel I hergestellt werden. Dieses Hydrolyse kann nach an sich bekannten Methoden, beispielsweise in Analogie zu Verfahrensvariante c), durchgeführt werden.

Die Isocyanate der Formel VIIIb können hergestellt werden, indem man eine Verbindung der Formel Ib, worin $Q^1$ die Gruppe der Formel -NH- bedeutet, in einem inerten Lösungsmittel mit Phosgen behandelt. Geeignete Lösungsmittel sind beispielsweise halogenierte Kohlenwasserstoffe, wie Chloroform und 1,2-Dichloräthan. Die Isocyanate der Formel VIIIb können aber auch hergestellt werden, indem man ein Carbonsäurehalogenid der Formel VIIIc in einem inerten organischen Lösungsmittel mit einem Azid, wie Natriumazid oder Trimethylsilylazid, in das entsprechende Carbonsäureazid überführt und dieses durch Erwärmen zum entsprechenden Isocyanat umlagert. Geeignete Lösungsmittel sind beispielsweise Aether, wie Dioxan, Aethylenglykoldimethyläther, Diäthylenglykoldimethyläther und dergleichen, Ketone, wie Aethylmethylketon, und dergleichen. Die Umlagerung wird bei Temperaturen von 80°C und darüber bewerkstelligt.

Die Carbonsäurehalogenide der Formel VIIIc können hergestellt werden, indem man eine Carbonsäure der Formel Ia mit einem Halogenierungsmittel behandelt. Geeignete Halogenierungsmittel sind beispielsweise Thionylchlorid, Oxalylchlorid, Phosphorpentachlorid und dergleichen. In einer bevorzugten Ausführungsform verwendet man überschüssiges Thionylchlorid und arbeitet ohne zusätzliches Lösungsmittel bei Raumtemperatur.

Die Diazoketone der Formel VIIId können hergestellt werden, indem man ein Carbonsäurehalogenid der Formel VIIIc in einem inerten organischen Lösungsmittel mit Diazomethan umsetzt. Geeignete Lösungsmittel sind beispielsweise Aether, wie Tetrahydrofuran, Dioxan und Diäthyläther. Die Reaktion wird vorzugsweise in einem Temperaturbereich von etwa 0° bis etwa 10°C durchgeführt.

Die Verbindungen der Formel VIIIe können in Analogie zur Verfahrensvariante a) hergestellt werden, wobei man als Ausgangsstoff eine Verbindung der allgemeinen Formel $HC{\equiv}C\text{-}CH(OR^7)OR^8$ verwendet, worin $R^7$ und $R^8$ obige Bedeutung besitzen.

Die Verbindungen der Formel VIIIf können hergestellt werden, indem man eine Verbindung der Formel Ib′ in einem inerten Lösungsmittel, beispielsweise in einem Aether, wie Dioxan, und in Gegenwart einer Base, beispielsweise eines basischen Amins, wie Pyridin, mit Chlorameisensäurephenylester umsetzt.

Die übrigen als Ausgangsstoffe verwendeten Verbindungen gehören an sich bekannten Stoffklassen an.

Wie bereits eingangs erwähnt besitzen die Verbindungen der Formel I wertvolle pharmakologische Eigenschaften. Sie entfalten insbesondere ausgeprägte muskelrelaxierende, sedativ-hypnotische, antikonvulsive und/oder anxiolytische Eigenschaften und weisen eine nur geringe Toxizität auf. Diese Eigenschaften können beispielsweise im nachfolgend beschriebenen und für die Erfassung derartiger Eigenschaften allgemein anerkannten Antipentetrazoltest nachgewiesen werden.

In diesem Tierversuch verabreicht man Ratten die zu testende Verbindung oral und 30 Minuten später 120 mg/kg Pentetrazol intraperitoneal, das in ungeschützten Versuchstieren 1-4 Minuten nach der Injektion Emprosthotonus und tonische Streckungen der vorderen und/oder hinteren Gliedmassen bewirkt. Man verwendet 10 Versuchstiere pro Dosis Testsubstanz. Nach Auszählen der geschützten Versuchstiere ermittlet man die $ED_{50}$ nach der Probit-Methode. Die $ED_{50}$ ist diejenire Dosis, welche 50% der Versuchstiere vor den durch Pentetrazol bewirkten krampfartigen Anfällen schützt. In der nachfolgenden Tabelle werden die Resultate zusammengestellt, welche mit repräsentativen Vertretern der durch die allgemeine Formal A definierten Verbindungsklasse in dem vorstehend geschilderten Versuch erhalten worden sind. Die Tabelle enthält ausserdem Angaben über die akute Toxizität ($DL_{50}$) dieser Verbindungen in mg/kg bei einmaliger oraler Verabreichung an Mäusen.

Tabelle

| Verbindung | $ED_{50}$ in mg/kg p.o. | $DL_{50}$ in mg/kg p.o. |
|---|---|---|
| A | 3,1 | >3000 |
| B | 0,44 | >3000 |
| C | 0,56 | - |
| D | 0,23 | - |
| E | 1,4 | - |
| F | 3,3 | 3000 |
| G | 8,2 | >5000 |
| H | 0,29 | >5000 |

A = 4-[(10-Chlor-6,7-dihydro-4-oxo-3-phenyl-4H-pyrido[2,1-a]-phthalazin-1-yl)carbonyl]morpholin

B = (R)-1-[(10-Chlor-6,7-dihydro-4-oxo-3-phenyl-4H-pyrido-[2,1-a]phthalazin-1-yl)carbonyl]-2-(methoxymethyl)-pyrrolidin

C = 1-[(10-Chlor-6,7-dihydro-4-oxo-3-phenyl-4H-pyrido[2,1-a]-phthalazin-1-yl)carbonyl]-3-methoxyazetidin

D = 10-Chlor-6,7-dihydro-N,N-dimethyl-4-oxo-3-phenyl-4H-pyrido[2,1-a]phthalazin-1-carboxamid

E = (R)-1-[(4,5-Dihydro-7-oxo-8-phenyl-7H-pyrido[1,2-b]-thieno[2,3-d]pyridazin-10-yl)carbonyl]-3-methoxy-pyrrolidin

F = 1-[(4,5-Dihydro-4-methyl-7-oxo-8-phenyl-7H-pyrido-[1,2-b]thieno[2,3-d]pyridazin-10-yl)carbonyl]-3-methoxy-azetidin

G = 1-[(4,5-Dihydro-5-methyl-7-oxo-8-phenyl-7H-pyrido-[1,2-b]thieno[2,3-d]pyridazin-10-yl)carbonyl]-3-methoxy-azetidin

H = 4,5-Dihydro-N,N-dimethyl-7-oxo-8-phenyl-7H-pyrido-[1,2-b]thieno[2,3-d]pyridazin-10-carboxamid.

Die Verbindungen der Formel A und die pharmazeutisch annehmbaren Säureadditionssalze von Verbindungen der Formel A mit einem basischen Substituenten können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten. Arzneimittel enthaltend ein erfindungsgemässes Produkt und einen therapeutisch inerten Träger, sowie ein Verfahren zu deren Herstellung, das dadurch gekennzeichnet ist, dass man ein erfindungsgemässes Produkt und gegebenenfalls einen oder mehrere andere therapeutisch wertvolle Stoffe in eine galenische Darreichungsform bringt, sind ebenfalls Gegenstand der vorliegenden Erfindung.

Die erfindungsgemässen Produkte können, wie bereits erwähnt, bei der Bekämpfung oder Verhütung von Krankheiten, insbesondere bei der Bekämpfung von Konvulsionen und Angstzuständen, sowie für die Herstellung von Arzneimitteln mit muskelrelaxierenden, sedativ-hypnotischen, antikonvulsiven und/oder anxiolytischen Eigenschaften verwendet werden. Die Dosierung kann dabei innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 1 mg bis etwa 100 mg.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

## Beispiel I

13,9 g 7-Nitro-1(2H)-phthalazinon und 16,3 g Lawesson-Reagenz in 170 ml Acetonitril werden während 45 Minuten unter Rückfluss erhitzt. Das Reaktionsgemisch wird heiss filtriert. Das Filtrat wird im Eisbad gekühlt, und die erhaltenen Kristalle werden abgesaugt. Nach Trocknen im Vakuum und Umkristallisieren erhält man 7-Nitro-1(2H)-phthalazin-thion als gelbe Kristalle vom Smp. 233-234° (Methanol/Acetonitril).

## Beispiel II

a) 66,95 g Thieno[2,3-d]pyridiazin-7(6H)-thion werden unter Argon in 3200 ml Methylenchlorid suspendiert, worauf man 102,7 g α-Bromphenylessigsäurechlorid dazutropft. Man rührt ca. 30 Minuten bei Raumtemperatur und tropft dann 121 ml Triäthylamin dazu, wobei auf 25-30° gekühlt wird. Es wird etwa 40 Minuten gerührt. Nach Abdampfen des Lösungsmittels im Vakuum wird der Rückstand in 1000 ml Wasser und 200 ml Aether aufgenommen, worauf man ca. 30 Minuten rührt. Die ausgefallenden Kristalle werden abgesaugt, mit Wasser und Aether gewaschen und über Nacht getrocknet. Die getrockneten Kristalle werden erneut mit 1000 ml Wasser verrührt. Nach dem Absaugen werden die rotvioletten Kristalle mit

Wasser gewaschen und im Vakuum getrocknet. Man erhält 3-Hydroxy-2-phenylthiazolo[3,2-b]thieno[2,3-d]-pyridazin-4-ium-hydroxid (inneres Salz) vom Smp. 260-264° (Zersetzung).

In analoger Weise erhält man:

b) Aus 7-Chlor-1(2H)-phthalazin-thion und α-Bromphenylessigsäurechlorid nach Umkristallisieren das 9-Chlor-3-hydroxy-2-phenylthiazolo[2,3-a]phthalazin-4-ium-hydroxid (inneres Salz) vom Smp. 296-298° (Chloroform);

c) aus 4-Methylthieno[2,3-d]pyridazin-7(6H)-thion und α-Bromphenylessigsäurechlorid das 7-Hydroxy-4-methyl-8-phenylthiazolo[3,2-b]thieno[2,3-d]pyridazin-6-ium hydroxid (inneres Salz) vom Smp. 294-296° (Chloroform).

## Beispiel III

a) 0,41 g 7-Nitro-1(2H)-phthalazin-thion und 0,37 g 3-Phenyl-2,2-oxirandicarbonitril in 10 ml Aceton werden während 30 Stunden unter Rückfluss erhitzt. Nach Abkühlen im Eisbad werden die erhaltenen violetten Kristalle abgesaugt, mit Aether gewaschen und im Vakuum getrocknet. Man erhält 9-Nitro-3-hydroxy-2-phenylthiazolo[2,3-a]phthalazin-4-ium hydroxid (inneres Salz) vom Smp. 303-305°.

In analoger Weise erhält man:

b) Aus 7-Chlor-1(2H)-phthalazin-thion und 3-(p-Chlorphenyl)-2,2-oxirandicarbonitril das 9-Chlor-2-(p-chlorphenyl)-3-hydroxythiazolo[2,3-a]phthalazinium hydroxid (inneres Salz) vom Smp. >300° (N,N-Dimethylformamid/Diäthyläther);

c) aus 7-Chlor-1(2H)-phthalazin-thion und 3-(m-Chlorphenyl)-2,2-oxirandicarbonitril das 9-Chlor-2-(m-chlorphenyl)-3-hydroxythiazolo[2,3-a]phthalazinium hydroxid (inneres Salz) vom Smp. 298-300° (N,N-Dimethylformamid);

d) aus 7-Chlor-1(2H)-phthalazin-thion und 3-(o-Chlorphenyl)-2,2-oxirandicarbonitril das 9-Chlor-2-(o-chlorphenyl)-3-hydroxythiazolo[2,3-a]phthalazinium hydroxid (inneres Salz) vom Smp. 289-291° (N,N-Dimethylformamid);

## Beispiel IV

a) 13,5 g 3-Hydroxy-2-phenylthiazolo[3,2-a]phthalazinium-hydroxid (inneres Salz) und 8,1 ml Propiolsäuremethylester werden in 200 ml Toluol unter Feuchtigkeitsausschluss während 24 Stunden unter Rückfluss erhitzt. Man lässt dann abkühlen und rührt während 1 Stunde im Eisbad. Die Kristalle werden abgesaugt, getrocknet und schliesslich aus Toluol umkristallisiert. Man erhält den 4-Oxo-3-phenyl-4H-pyrido[2,1-a]phthalazin -1-carbonsäuremethylester als gelbe Kristalle vom Smp. 174-175°.

In analoger Weise erhält man:

b) Aus 3-Hydroxy-2-phenylthiazolo[3,2-b]thieno[2,3-d]pyridazin-4-ium-hydroxid (inneres Salz) und Propiolsäure methylester nach Umkristallisieren aus Acetonitril den 7-Oxo-8-phenyl-7H-pyrido[1,2-b]thieno-[2,3-d]pyridazin-10-carbonsäuremethylester als gelbe Kristalle vom Smp. 198-199°;

c) aus 9-Chlor-3-hydroxy-2-phenylthiazolo[2,3-a]phthalazin-4-ium-hydroxid (inneres Salz) und Propiolsäuremethylester nach Umkristallisieren aus Acetonritril den 10-Chlor-4-oxo-3-phenyl-4H-pyrido[2,1-a]-phthalazin-1-carbonsäuremethylester als gelbe Kristalle vom Smp. 234-236°;

d) aus 9-Nitro-3-hydroxy-2-phenylthiazolo[2,3-a]phthalazin-4-ium-hydroxid (inneres Salz) und Propiolsäuremethylester den10-Nitro-4-oxo-3-phenyl-4H-pyrido[2,1-a]phthalazin-1-carbonsäuremethylester vom Smp. 227-230° (N,N-Dimethylformamid/Methanol);

e) aus 9-Chlor-2-(p-chlorphenyl)-3-hydroxythiazolo[2,3-a]phthalazinium hydroxid (inneres Salz) und Propiolsäuremethylester den 10-Chlor-3-(p-chlorphenyl)-4-oxo-4H-pyrido[2,1-a]phthalazin-1-carbonsäuremethylester vom Smp. 233-237° (2-Propanol/N,N-Dimethylformamid);

f) aus 7-Hydroxy-4-methyl-8-phenylthiazolo[3,2-b]thieno[2,3-d]pyridazin-6-ium hydroxid (inneres Salz) und Propiolsäuremethylester den 4-Methyl-7-oxo-8-phenyl-7H-pyrido[1,2-b]thieno[2,3-d]pyridazin-10-carbonsäuremethylester vom Smp. 183-184° (Essigester);

g) aus 9-Chlor-2-(m-chlorphenyl)-3-hydroxythiazolo[2,3-a]phthalazinium hydroxid (inneres Salz) und Propiolsäuremethylester den 10-Chlor-3-(m-chlorphenyl)-4-oxo-4H-pyrido[2,1-a]phthalazin-1-carbonsäuremethylester vom Smp. 234-235° (N,N-Dimethylformamid/Methanol);

h) aus 9-Chlor-2-(o-chlorphenyl)-3-hydroxythiazolo[2,3-a]phthalazinium hydroxid (inneres Salz) und Propiolsäure methylester den 10-Chlor-3-(o-chlorphenyl)-4-oxo-4H-pyrido[2,1-a]phthalazin-1-carbonsäuremethylester vom Smp. 220-222° (Acetonitril).

## Beispiel V

a) 11 g 4-Oxo-3-phenyl-4H-pyrido[2,1-a]phthalazin -1-carbonsäuremethylester werden unter Argon in 300 ml Aethanol aufgenommen, worauf man eine Lösung von 3,7 g Kaliumhydroxid in 30 ml Wasser hinzufügt und unter Rückfluss erhitzt, bis die Reaktion beendet ist. Dann wird das Reaktionsgemisch auf Zimmertemperatur abgekühlt und auf 2200 ml Wasser gegossen. Man stellt durch Zugabe von 1N wässeriger Salzsäure auf pH 7 und entfernt Verunreinigungen durch zweimalige Extraktion mit je 300 ml Methylenchlorid. Die Wasserphase wird mit 2N wässeri- ger Salzsäure auf pH 1 angesäuert, und die gebildeten Kristalle werden abgesaugt. Nach mehrmaligem Waschen mit Wasser und Trocknen im Vakuum erhält man 4-Oxo-3-phenyl-4H-pyrido[2,1-a]phthalazin-1-carbonsäure als gelbe Kristalle vom Smp. 236-237° (Zers.).

In analoger Weise erhält man:

b) Aus 7-Oxo-8-phenyl-7H-pyrido[1,2-b]thieno[2,3-d]phthalazin-1-carbonsäuremethylester nach Umkristallisieren aus Dimethylformamid die 7-Oxo-8-phenyl-7H-pyrido[1,2-b]thieno[2,3-d]pyridazin-10-carbonsäure als gelbe Kristalle vom Smp. 262-264° (Zersetzung);

c) aus 10-Chlor-4-oxo-3-phenyl-4H-pyrido[2,1-a]phthalazin-1-carbonsäuremethylester nach Umkristallisieren aus Acetonitril/Dimethylformamid die 10-Chlor-4-oxo-3-phenyl-4H-pyrido[2,1-a]phthalazin-1-carbonsäure als gelbe Kristalle vom Smp. 242° (Zersetzung);

d) aus 4-Methyl-7-oxo-8-phenyl-7H-pyrido[1,2-b]thieno[2,3-d]pyridazin-10-carbonsäuremethylester die 4-Methyl -7-oxo-8-phenyl-7H-pyrido[1,2-b]thieno[2,3-d]pyridazin-10-carbonsäure vom Smp. 243-245° (Acetonitril/N,N-Dimethylformamid).

## Beispiel VI

a) 3,68 g 4-Oxo-3-phenyl-4H-pyrido[2,1-a]phthalazin-1-carbonsäure werden unter Feuchtigkeitsausschluss in 80 ml Toluol suspendiert, worauf man 5,1 ml Thionylchlorid und 0,2 ml N,N-Dimethylformamid dazugibt und etwa während 2 Stunden bei Zimmertemperatur rührt. Die Reaktionsmischung wird im Vakuum eingedampft; der Rückstand wird in 50 ml Toluol aufgenommen, worauf man erneut im Vakuum eindampft. Das so erhaltene reine Carbonsäurechlorid wird in 90 ml Dioxan aufgenommen, worauf man nacheinander 6,52 ml Triäthylamin und 1,4 g 3-Hydroxyazetidin-hydrochlorid dazugibt. Es wird bis zur Beendigung der Reaktion bei Zimmertemperatur gerührt. Nach Entfernen des Lösungsmittels im Vakuum wird der Rückstand mit einem Gemisch aus 200 ml Wasser und 100 ml gesättigter wässeriger Kochsalzlösung versetzt, worauf man auf ca. 2° abkühlt und während 30 Minuten verrührt. Die Kristalle werden abgesaugt und zweimal mit 15 ml Wasser gewaschen. Die so erhaltenen Kristalle werden im Vakuum bei 70° getrocknet. Die Wasserphase wird dreimal mit Methylenchlorid extrahiert; die vereinigten organischen Phasen werden einmal mit 50 ml Wasser gewaschen, über Natriumsulfat getrocknet, filtriert und eingedampft. Die Kristalle werden mit dem oben erhaltenen Material vereinigt und in 150 ml Aether während 30 Minuten verrührt. Der Aether wird durch Filtration entfernt, und die gelben Kristalle werden getrocknet. Man erhält 3-Hydroxy-1-[(4-oxo-3-phenyl-4H-pyrido[2,1-a]phthalazin-1-yl)carbonyl]azetidin vom Smp. 260-264° (Zers.).

In analoger Weise erhält man aus 4-Oxo-3-phenyl-4H-pyrido[2,1-a]phthalazin-1-carbonsäure und:

b) Morpholin das 4-[(4-Oxo-3-phenyl-4H-pyrido[2,1-a]phthalazin-1-yl)carbonyl]morpholin vom Smp. 246-248° (Acetonitril);

c) R)-2-(Methoxymethyl)-pyrrolidin das (R)-2-(Methoxymethyl)-1-[(4-oxo-3-phenyl-4H-pyrido[2,1-a]-phthalazin-1-yl)carbonyl]pyrrolidin vom Smp. 187-189° (Essigester);

d) Dimethylamin das N,N-Dimethyl-4-oxo-3-phenyl-4H-pyrido[2,1-a]phthalazin-1-carboxamid vom Smp. 239-240° (Aethanol);

e) (R)-3-Methoxy-pyrrolidin das (R)-3-Methoxy-1-[(4-oxo-3-phenyl-4H-pyrido[2,1-a]phthalazin-1-yl)-carbonyl]pyrrolidin vom Smp. 208-209° (Aethanol);

f) (S)-3-Methoxy-pyrrolidin das (S)-3-Methoxy-1-[(4-oxo-3-phenyl-4H-pyrido[2,1-a]phthalazin-1-yl)-carbonyl]pyrrolidin vom Smp. 205-206° (Acetonitril).

In analoger Weise erhält man aus 10-Chlor-4-oxo-3-phenyl-4H-pyrido[2,1-a]phthalazin-1-carbonsäure und:

g) N-Aethyl-N-(2-methoxyäthyl)amin das N-Aethyl-N-(2-methoxyäthyl)-10-chlor-4-oxo-3-phenyl-4H-pyrido[2,1-a]phthalazin-1-carboxamid vom Smp. 167° (Essigester);

h) 3-Methoxyazetidin das 1-[(10-Chlor-4-oxo-3-phenyl-4H-pyrido[2,1-a]phthalazin-1-yl)carbonyl]-3-methoxyazetidin vom Smp. 238-240° (Acetonitril);

i) Dimethylamin das N,N-Dimethyl-10-chlor-4-oxo-3-phenyl-4H-pyrido[2,1-a]phthalazin-1-carboxamid vom Smp. 244-246° (Aethanol);

j) (R)-3-Methoxypyrrolidin das (R)-1-[(10-Chlor-4-oxo-3-phenyl-4H-pyrido[2,1-a]phthalazin-1-yl)-carbonyl]-3-methoxypyrrolidin vom Smp. 203-206° (Acetonitril);

k) (S)-3-Methoxypyrrolidin das (S)-1-[(10-Chlor-4-oxo-3-phenyl-4H-pyrido[2,1-a]phthalazin-1-yl)-carbonyl]-3-methoxypyrrolidin vom Smp. 204-206° (Acetonitril);

l) (S)-Prolinol den (S)-1-[(10-Chlor-4-oxo-3-phenyl-4Hpyrido[2,1-a]phthalazin-1-yl)carbonyl]-2-pyrrolidinmethanol vom Smp. 245-255° (Zers.; Acetonitril).

In analoger Weise erhält man aus 4-Methyl-7-oxo-8-phenyl-7H-[pyrido[1,2-b]thieno[2,3-d]pyridazin-10-carbonsäure und:

m) Dimethylamin das N,N,4-Trimethyl-7-oxo-8-phenyl-7H-pyrido[1,2-b]thieno[2,3-d]pyridazin-10-carboxamid vom Smp. 244-246° (Aethanol);

n) 3-Methoxyazetidin das 3-Methoxy-1-[(4-methyl-7-oxo-8-phenyl-7H-pyrido[1,2-b]thieno[2,3-d]-pyridazin-10-yl)carbonyl]azetidin vom Smp. 240-241° (Aethanol);

o) (R)-2-(Methoxymethyl)-pyrrolidin das (R)-2-(Methoxymethyl)-1-[(4-methyl-7-oxo-8-phenyl-7H-pyrido[1,2-b]thieno[2,3-d]pyridazin-10-yl)carbonyl]pyrrolidin vom Smp. 209-212° (Aethanol);

p) (R)-Prolinol den (R)-1-[(4-Methyl-7-oxo-8-phenyl-7H-pyrido[1,2-b]thieno[2,3-d]pyridazin-10-yl)-carbonyl]-2-pyrrolidinmethanol vom Smp. 271-274° (Aethanol).

## Beispiel 1

a) 1,26 g 1-[(10-Chlor-4-oxo-3-phenyl-4H-pyrido[2,1-a]phthalazin-1-yl)carbonyl]-3-methoxyazetidin und 0,75 g Natriumcyanoborhydrid werden in 30 ml Methanol unter Argon tropfenweise mit gesättigter methanolischer Salzsäure bei Raumtemperatur versetzt. Nach kurzer Zeit ist die Reaktion beendet. Es wird auf 70 ml Eiswasser gegossen, und die gelblichen Kristalle werden abfiltriert. Man wäscht dreimal mit je 2 ml Wasser und trocknet bei 60° im Vakuum. Nach Umkristallisieren des Rohproduktes aus Essigsäureäthylester/Aceton (1:1) erhält man 1-[(10-Chlor-6,7-dihydro-4-oxo-3-phenyl-4H-pyrido[2,1-a]-phthalazin-1-yl)carbonyl]-3-methoxyazetidin als gelbliche Kristalle vom Smp. 238-239°.

Anstelle von Methnol kann auch ein Gemisch aus Tetrahydrofuran und Essigsäure als Lösungsmittel verwendet werden, wobei in diesem Fall keine methanolische Salzsäure dazugegeben werden muss.

In analoger Weise erhält man:

b) Aus N-Aethyl-10-chlor-N-(2-methoxyäthyl)-4-oxo-3-phenyl-4H-pyrido[2,1-a]phthalazin-1-carboxamid in Methanol/Eisessig (10:1), jedoch ohne Zusatz von gesättigter methanolischer Salzsäure, das N-Aethyl-10-chlor-6,7-dihydro-N-(2-methoxyäthyl)-4-oxo-3-phenyl-4H-pyrido[2,1-a]phthalazin-1-carboxamid vom Smp. 184-185° (Essigsäureäthylester);

c) aus 10-Chlor-N,N-dimethyl-4-oxo-3-phenyl-4H-pyrido[2,1-a]phthalazin-1-carboxamid in Methanol/Esessig (10:1), jedoch ohne Zusatz von gesättigter methanolischer Salzsäure, das 10-Chlor-6,7-dihydro-N,N-dimethyl-4-oxo-3-phenyl-4H-pyrido[2,1-a]phthalazin-1-carboxamid vom Smp. 250-252° (Aethanol);

d) aus 4-[(4-Oxo-3-phenyl-4H-pyrido[2,1-a]phthalazin-1-yl)carbonyl]morpholin in Methanol/Eisessig (10:1), jedoch ohne Zusatz von gesättigter methanolischer Salzsäure, das 4-[(6,7-Dihydro-4-oxo-3-phenyl-4H-pyrido[2,1-a]phthalazin-1-yl)carbonyl]morpholin vom Smp. 254-257° (Acetonitril);

e) aus 7-Oxo-8-phenyl-7H-pyrido[1,2-b]thieno[2,3-d]pyridazin-10-carbonsäuremethylester in Essigsäure den 4,5-Dihydro-7-oxo-8-phenyl-7H-pyrido[1,2-b]thieno[2,3-d]pyridazin-10 -carbonsäuremethylester vom Smp. 218° (Acetonitril);

f) aus (R)-1-[(10-Chlor-4-oxo-3-phenyl-4H-pyrido[2,1-a]phthalazin-1-yl)carbonyl]-2-(methoxymethyl)-pyrrolidin das (R)-1-[(10-Chlor-6,7-dihydro-4-oxo-3-phenyl-4H-pyrido[2,1-a]phthalazin-1-yl)carbonyl]-2-(methoxymethyl)pyrrolidin vom Smp. 161-164° (Essigester/Diäthyläther);

g) aus 4-[(10-Chlor-4-oxo-3-phenyl-4H-pyrido[2,1-a]phthalazin-1-yl)carbonyl]morpholin das 4-(10-Chlor-6,7-dihydro-4-oxo-3-phenyl-4H-pyrido[2,1-a]phthalazin-1-yl)carbonyl]morpholin vom Smp. 256-258° (Acetonitril);

h) aus (R)-1-[(10-Chlor-4-oxo-3-phenyl-4H-pyrido[2,1-a]phthalazin-1-yl)carbonyl]-3-methoxypyrrolidin das (R)-1-[(10-Chlor-6,7-dihydro-4-oxo-3-phenyl-4H-pyrido[2,1-a]phthalazin-1-yl)carbonyl]-3-methoxypyrrolidin vom Smp. 180-181° (Acetonitril);

i) aus (S)-1-[(10-Chlor-4-oxo-3-phenyl-4H-pyrido[2,1-a]phthalazin-1-yl)carbonyl]-3-methoxypyrrolidin das (S)-1-[(10-Chlor-6,7-dihydro-4-oxo-3-phenyl-4H-pyrido[2,1-a]phthalazin-1-yl)carbonyl]-3-methoxypyrrolidin vom Smp. 179-181° (Acetonitril);

j) aus (R)-[(7-Oxo-8-phenyl-7H-pyrido[1,2-b]thieno[2,3-d]pyridazin-10-yl)carbonyl]-2-pyrrolidinmethanol den (R)-1-[(4,5-Dihydro-7-oxo-8-phenyl-7H-pyrido[1,2-b]thieno[2,3-d]pyridazin-10-yl)carbonyl]-2-pyrrolidinmethanol vom Smp. 218-219° (Methanol);

k) aus N-Aethyl-N-(2-methoxyäthyl)-7-oxo-8-phenyl-7H-pyrido[1,2-b]thieno[2,3-d]pyridazin-10-carboxamid das N-Aethyl-4,5-dihydro-N-(2-methoxyäthyl)-7-oxo-8-phenyl-7H-pyrido[1,2-b]thieno[2,3-d]pyridazin-10-carboxamid vom Smp. 148-149° (Essigester);

l) aus N-(3-Methoxypropyl)-7-oxo-8-phenyl-7H-pyrido[1,2-b]thieno[2,3-d]pyridazin-10-carboxamid das 4,5-Dihydro-N-(3-methoxypropyl)-7-oxo-8-phenyl-7H-pyrido[1,2-b]thieno[2,3-d]pyridazin-10-carboxamid vom Smp. 220-221° (Acetonitril);

m) aus (R)-3-Methoxy-(7-oxo-8-phenyl-7H-pyrido[1,2-b]thieno[2,3-d]pyridazin-10-yl)carbonyl]pyrrolidin das (R)-1-[(4,5-Dihydro-7-oxo-8-phenyl-7H-pyrido[1,2-b]thieno[2,3-d]pyridazin-10-yl)carbonyl]-3-methoxypyrrolidin vom Smp. 160-161° (Essigester);

n) aus 4-[(7-Oxo-8-phenyl-7H-pyrido[1,2-b]thieno[2,3-d]pyridazin-10-yl)carbonyl]morpholin das 4-[(4,5-Dihydro-7-oxo-8-phenyl-7H-pyrido[1,2-b]thieno[2,3-d]pyridazin-10-yl)carbonyl]morpholin vom Smp. 275-278° (Acetonitril);

o) aus 3-Methoxy-1-[(4-methyl-7-oxo-8-phenyl-7H-pyrido[1,2-b]thieno[2,3-d]pyridazin-10-yl)carbonyl]-azetidin das rac-1-[(4,5-Dihydro-4-methyl-7-oxo-8-phenyl-7H-pyrido[1,2-b]thieno[2,3-d]pyridazin-10-yl)-carbonyl]-3-methoxyazetidin vom Smp. 169-170° (Aethanol);

p) aus 3-Methoxy-1-[(7-oxo-8-phenyl-7H-pyrido[1,2-b]thieno[2,3-d]pyridazin-10-yl)carbonyl]azetidin das 1-[(4,5-Dihydro-7-oxo-8-phenyl-7H-pyrido[1,2-b]thieno[2,3-d]pyridazin-10-yl)carbonyl]-3-methoxyazetidin vom Smp. 254-255° (N,N-Dimethylformamid/Methanol);

q) aus N,N-Diäthyl-7-oxo-8-phenyl-7H-pyrido[1,2-b]thieno[2,3-d]pyridazin-10-yl)carbonyl]azetidin das N,N-Diäthyl-4,5-dihydro-7-oxo-8-phenyl-7H-pyrido[1,2-b]thieno[2,3-d]pyridazin-10-carboxamid vom Smp. vom 179-180° (Essigester);

r) aus N,N-Dimethyl-7-oxo-8-phenyl-7H-pyrido[1,2-b]thieno[2,3-d]pyridazin-10-yl)carboxamid das 4,5-Dihydro-N,N-dimethyl-7-oxo-8-phenyl-7H-pyrido[1,2-b]thieno[2,3-d]pyridazin -10-carboxamid vom Smp. vom 246-250° (Aethanol/N,N-Dimethylformamid);

s) aus (S)-2-Methoxymethyl-1-[(7-oxo-8-phenyl-7H-pyrido[1,2-b]thieno[2,3-d]pyridazin-10-yl)carbonyl]-pyrrolidin das (S)-1-(4,5-Dihdyro-7-oxo-8-phenyl-7H-pyrido[1,2-b]thieno[2,3-d]pyridazin-10-yl)carbonyl]-2-(methoxymethyl)pyrrolidin vom Smp. 172-173° (Aethanol);

t) aus (R)-2-(Methoxymethyl)-1-[(7-oxo-8-phenyl-7H-pyrido[1,2-b]thieno[2,3-d]pyridazin-10-yl)-carbonyl]pyrrolidin das (R)-1-(4,5-Dihdyro-7-oxo-8-phenyl-7H-pyrido[1,2-b]thieno[2,3-d]pyridazin-10-yl)-carbonyl]-2-(methoxymethyl)pyrrolidin vom Smp. 170-172° (Aethanol);

u) aus N,N,4-Trimethyl-7-oxo-8-phenyl-7H-pyrido[1,2-b]thieno[2,3-d]pyridazin-10-carboxamid das 4,5-Dihydro-N,N,4-trimethyl-7-oxo-8-phenyl-7H-pyrdio[1,2-b]thieno[2,3-d]pyridazin-10-carboxamid vom Smp. 172-174° (Toluol);

v) aus 3-Methoxy-1-[(4-oxo-3-phenyl-4H-pyrido[2,1-a]phthalazin-1-yl)carbonyl]azetidin das 1-[(6,7-Dihydro-4-oxo-3-phenyl-4H-pyrido[2,1-a]phthalazin-1-yl)carbonyl]-3-methoxyazetidin vom Smp. 196-197° (Aethanol).

## Beispiel 2

a) 2,7 g 10-Chlor-N,n-diäthyl-4-oxo-3-phenyl-4H-pyrido[2,1-a]phthalazin-1-carboxamid werden in 65 ml Tetrahydrofuran unter Argon suspendiert, worauf man 150 mg Lithiumborhydrid dazugibt, während 4 Stunden bei Raumtemperatur rührt, nochmals 75 mg Lithiumborhydrid dazugibt und bis zur Beendigung der Reaktion weiterrührt. Die Reaktionsmischung wird auf 300 ml Eiswasser gegossen, wobei das Produkt auskristallisiert. Das Rohprodukt wird an Kieselgel chromatographiert. Durch Umkristallisieren aus Toluol

erhält man 10-Chlor-N,N-diäthyl-6,7-dihydro-4-oxo-4-phenyl-4H-pyrido [2,1-a]phthalazin-1-carboxamid vom Smp. 192-194° (Toluol).

In analoger Weise erhält man:

b) Aus (S)-1-[(10-Chlor-4-oxo-3-phenyl-4H-pyrido[2,1-a]phthalazin-1-yl)carbonyl]-2-(methoxymethyl)-pyrrolidin das (S)-1-[(10-Chlor-6,7-dihydro-4-oxo-3-phenyl-4H-pyrido[2,1-a]phthalazin-1-yl)carbonyl]-2-(methoxymethyl)pyrrolidin vom Smp. 166-167° (Essigester);

c) aus (R)-1-[(4-Methyl-7-oxo-8-phenyl-7H-pyrido[1,2-b]thieno[2,3-d]pyridazin-10-yl)carbonyl]-2-(methoxymethyl)pyrrolidin das (R)-1-[(4,5-Dihydro-4-methyl-7-oxo-8-phenyl-7H-pyrido[1,2-b]thieno[2,3-d]-pyridazin-10-yl)carbonyl]-2-(methoxymethyl)pyrrolidin vom Smp. 150-155° (Toluol/Diäthyläther);

d) aus (S)-1-[(10-Chlor-4-oxo-3-phenyl-4H-pyrido[2,1-a]phthalazin-1-yl)carbonyl]-2-pyrrolidinmethanol das (S)-1-[(10-Chlor-6,7-dihydro-4-oxo-3-phenyl-4H-pyrido[2,1-a]phthalazin-1-yl)carbonyl]-2-pyrrolidinmethanol vom Smp. 222-225° (Methanol/N,N-Dimethylformamid);

e) aus N,N-Dimethyl-4-oxo-3-phenyl-4H-pyrido[2,1-a]phthalazin-1-carboxamid das 6,7-Dihydro-N,N-dimethyl-4-oxo-3-phenyl-4H-pyrido[2,1-a]phthalazin-1-carboxamid vom Smp. 266-268° (N,N-Dimethylformamid/Aethanol);

f) aus (R)-3-Methoxy-1-(4-oxo-3-phenyl-4H-pyrido[2,1-a]phthalazin-1-yl)carbonyl]pyrrolidin das (R)-1-[-(6,7-Dihydro-4-oxo-3-phenyl-4H-pyrido[2,1-a]pthalazin-1-yl)carbonyl]-3-methoxypyrrolidin vom Smp. 175-176° (Toluol);

g) aus (S)-3-Methoxy-1-[(4-oxo-3-phenyl-4H-pyrido[2,1-a]phthalazin-1-yl)carbonyl]pyrrolidin das (S)-1-[(6,7-Dihydro-4-oxo-3-phenyl-4H-pyrido[2,1-a]phthalazin-1-yl)carbonyl]-3-methoxypyrrolidin vom Smp. 174-175° (Toluol);

h) aus 10-(3-Cyclopropyl-1,2,4-oxoadiazol-5-yl)-8-phenyl-7H-pyrido[1,2-b]thieno[2,3-d]pyridazin-7-on das 10-(3-Cyclopropyl-1,2,4-oxadiazol-5-yl)-4,5-dihydro-7-oxo-8-phenyl-7H-pyrido[1,2-b]thieno[2,3-d]-pyridazin vom Smp. 202-204° (Toluol);

i) aus 10-Nitro-4-oxo-3-phenyl-4H-pyrido[2,1-a]phthalazin-1-carbonsäuremethylester den 6,7-Dihydro-10-nitro-4-oxo-3-phenyl-4H-pyrido[2,1-a]phthalazin-1-carbonsäuremethylester vom Smp. 232-233° (N,N-Dimethylformamid);

j) aus 7-Oxo-8-phenyl-7H-pyrido[1,2-b]thieno[2,3-d]pyridazin-10-carbonsäuremethylester das 4,5-Dihydro-10-(hydroxymethyl)-8-phenyl-7H-pyrido[1,2-b]thieno[2,3-d]pyridazin-7-on vom Smp. 205-206° (Acetonitril).

## Beispiel 3

a) 0,34 g 7-Oxo-8-phenyl-7H-pyrido[1,2-b]thieno[2,3-d]pyridazin-10-carbonsäuremethylester werden in 10 ml Ameisensäure unter Argon bei 25-30° tropfenweise mit 0,31 g Natriumcyanoborhydrid in 2 ml Tetrahydrofuran versetzt. Man lässt bei Raumtemperatur während 24 Stunden rühren. Die Lösung wird auf 50 ml Eiswasser gegossen. Die gelblichen Kristalle werden abfiltriert und mit Wasser gewaschen. Nach Trocknen im Vakuum wird aus Essigester umkristallisiert. Man erhält 4,5-Dihydro-5-methyl-7-oxo-8-phenyl-7H-pyrido[1,2-b]thieno[2,3-d]pyridazin-10-carbonsäuremethylester vom Smp. 180-181°.

In analoger Weise erhält man:

b) Aus 3-Methoxy-1-[(7-oxo-8-phenyl-7H-pyrido[1,2-b]thieno[2,3-d]pyridazin-10-yl)carbonyl]azetidin das 1-[(4,5-Dihydro-5-methyl-7-oxo-8-phenyl-7H-pyrido[1,2-b]thieno[2,3-d]pyridazin-10-yl)carbonyl]-3-methoxyazetidin vom Smp. 221-222° (Methanol);

c) aus N,N-Dimethyl-7-oxo-8-phenyl-7H-pyrido[1,2-b]thieno[2,3-d]pyridazin-10-carboxamid das 4,5-Dihydro-N,N,5-trimethyl-7-oxo-8-phenyl-7H-pyrido[1,2-b]thieno[2,3-d]pyridazin-10-carboxamid vom Smp. 247-249° (Methanol);

d) aus (R)-2-(Methoxymethyl)-1-(7-oxo-8-phenyl-7H-pyrido[1,2-b]thieno[2,3-d]pyridazin-10-yl)carbonyl-[pyrrolidin das (R)-1-[(4,5-Dihydro-5-methyl-7-oxo-8-phenyl-7H-pyrido[1,2-b]thieno[2,3-d]pyridazin-10-yl)-carbonyl]-2-(methoxymethyl)pyrrolidin vom Smp. 204-207° (Aethanol);

e) aus (S)-1-[(7-Oxo-8-phenyl-7H-pyrido[1,2-b]thieno[2,3-d]pyridazin-10-yl)carbonyl]-2-(methoxymethyl)pyrrolidin das (S)-1-[(4,5-Dihydro-5-methyl-7-oxo-8-phenyl-7H-pyrido[1,2-b]thieno[2,3-d]-pyridazin-10-yl)carbonyl]-2-(methoxymethyl)pyrrolidin vom Smp. 208-209° (Aethanol);

f) aus N,N,4-Trimethyl-7-oxo-8-phenyl-7H-pyrido[1,2-b]thieno[2,3-d]pyridazin-10-carboxamid das 4,5-Dihydro-N,N,4,5-tetramethyl-7-oxo-8-phenyl-7H-pyrido[1,2-b]thieno[2,3-d]pyridazin-10-carboxamid vom Smp. 189-191° (Toluol);

g) aus (R)-2-(Methoxymethyl)-1-[(4-methyl-7-oxo-8-phenyl-7H-pyrido[1,2-b]thieno[2,3-d]pyridazin-10-yl)carbonyl]pyrrolidin das (R)-1-[(4,5-Dihydro-4,5-dimethyl-7-oxo-8-phenyl-7H-pyrido[1,2-b]thieno[2,3-d]-pyridazin-10-yl)carbonyl]-2-(methoxymethyl)pyrrolidin vom Smp. 155-157° (Essigester/Diäthyläther;

h) aus 3-Methoxy-1-[(4-oxo-3-phenyl-4H-pyrido[2,1-a]phthalazin-1-yl)carbonyl]azetidin das 1-[(6,7-Dihydro-6-methyl-4-oxo-3-phenyl-4H-pyrido[2,1-a]phthalazin-1-yl)carbonyl]-3-methoxyazetidin vom Smp. 226-227° (Toluol);

i) aus (R)-2-Methoxymethyl-1-[(4-oxo-3-phenyl-4H-pyrido[2,1-a]phthalazin-1-yl)carbonyl]pyrrolidin das (R)-1-[(6,7-Dihydro-6-methyl-4-oxo-3-phenyl-4H-pyrido[2,1-a]pthalazin -1-yl)carbonyl]-2-(methoxymethyl)-pyrrolidin vom Smp. 158-159° (Essigester/Diäthyläther);

j) aus N,N-Dimethyl-4-oxo-3-phenyl-4H-pyrido[2,1-a]pthalazin1-1carboxamid das 6,7-Dihydro-N,N,6-trimethyl-4-oxo-3-phenyl-4H-pyrido[2,1-a]phthalazin-1-carboxamid vom Smp. 131-132° (Acetonitril);

k) aus 4-[(10-Chlor-4-oxo-3-phenyl-4H-pyrido[2,1-a]phthalazin-1-yl)carbonyl]morpholin das 4-[(10-Chlor-6,7-dihydro-6-methyl-4-oxo-3-phenyl-4H-pyrido[2,1-a]pyridazin-1-yl)carbonyl]morpholin vom Smp. 282-284° (Acetonitril);

l) aus 10-Chlor-N,N-diäthyl-4-oxo-3-phenyl-4H-pyrido[2,1-a]phthalazin-1-carboxamid das 10-Chlor-N,N-diäthyl-6,7-dihydro-6-methyl-4-oxo-3-phenyl-4H-pyrido[2,1-a]phthalazin-1-carboxamid vom Smp. 187-188° (Toluol);

m) aus 10-Chlor-4-oxo-3-phenyl-N,N-dimethyl-4H-pyrido[2,1-a]phthalazin-1-carboxamid das 10-Chlor-6,7-dihydro-3-phenyl-N,N,6-trimethyl-4H-pyrido[2,1-a]phthalazin-1-carboxamid vom Smp. 231-233° (Acetonitril).

## Beispiel 4

0,54 g 4,5-Dihydro-7-oxo-8-phenyl-7H-pyrido[1,2-b]thieno[2,3-d]pyridazin-10-carbonsäuremethylester werden in 16 ml Essigsäureanhydrid suspendiert und während 3 Stunden auf etwa 100° erhitzt. Die Reaktionslösung wird im Vakuum eingedampft, und der Rückstand wird in 32 ml Wasser aufgenommen und verrührt. Es wird zweimal mit je 30 ml und einmal mit 15 ml Dichlormethan extrahiert. Nach Trocknen über Natriumsulfat wird filtriert und im Vakuum eingedampft. Der Rückstand wird an Kieselgel mit Dichlormethan/Aceton (9:1) chromatographiert. Durch Umkristallisieren aus Aether erhält man den 5-Acetyl-4,5-dihydro-7-oxo-8-phenyl-7H-pyrido[1,2-b]thieno[2,3-d]pyridazin-10-carbonsäuremethylester vom Smp. 132-134°.

## Beispiel 5

In Analogie zu den Beispielen V, VI und 2 erhält man:

a) Aus 10-Chlor-3-(p-chlorphenyl)-4-oxo-4H-pyrido[2,1-a]phthalazin-1-carbonsäuremethylester das 4-[-(10-Chlor-3-(p-chlorphenyl)-6,7-dihydro-4-oxo-4H-pyrido[2,1-a]phthalazin-1-yl)carbonyl]morpholin vom Smp. 238-240° (Acetonitril);

b) aus 10-Chlor-3-(m-chlorphenyl)-4-oxo-4H-pyrido[2,1-a]phthalazin-1-carbonsäuremethylester das 4-[(10-Chlor-3-(m-chlorphenyl)-6,7-dihydro-4-oxo-4H-pyrido[2,1-a]phthalazin-1-yl)carbonyl]morpholin vom Smp. 256-259° (N,N-Dimethylformamid/Methanol);

c) aus 10-Chlor-3-(o-chlorphenyl)-4-oxo-4H-pyrido[2,1-a]phthalazin-1-carbonsäuremethylester das 4-[-(10-Chlor-3-(o-chlorphenyl)-6,7-dihydro-4-oxo-4H-pyrido[2,1-a]phthalazin-1-yl)carbonyl]morpholin vom Smp. 200-202° (Essigester).

## Beispiel 6

a) 1,26 g 4-[(10-Chlor-6,7-dihydro-4-oxo-3-phenyl-4H-pyrido[2,1-a]phthalazin-1-yl)carbonyl]morpholin werden in 30 ml Dichlormethan unter Agon gelöst, worauf man 1,01 g Tetrabutylammoniumhydrogensulfat, 1,4 g Aethyljodid und 1,45 ml 50-proz. Natriumhydroxidlösung dazugibt und bis zur Beendigung der Reaktion bei Raumtemperatur rührt. Es werden 3 ml Wasser dazugegeben, die organische Phase wird abgetrennt und getrocknet, und das Lösungsmittel wird im Vakuum entfernt. Der Rückstand wird an

Kieselgel chromatographiert. Man erhält nach Umkristallisieren 4-[(6-Aethyl-10-chlor-6,7-dihydro-4-oxo-3-phenyl-4H-pyrido[2,1-a]phthalazin-1-yl)carbonyl]morpholin als gelbe Kristalle vom Smp. 288-289° (Acetonitril).

In analoger Weise erhält man aus 4-[(10-Chlor-6,7-dihydro-4-oxo-3-phenyl-4H-pyrido[2,1-a]phthalazin-1-yl)carbonyl]morpholin und:

b) Benzylchlorid das 4-[(6-Benzyl-10-chlor-6,7-dihydro-4-oxo-3-phenyl-4H-pyrido[2,1-a]phthalazin-1-yl)carbonyl]morpholin vom Smp. 180-183° (Toluol/Diäthyläther).

## Beispiel 7

2,62 g 4,5-Dihydro-10-(hydroxymethyl)-8-phenyl-7H-pyrido[1,2-b]thieno[2,3-d]pyridazin-7-on werden in 80 ml N,N-Dimethylformamid gelöst und in einem Eisbad auf 3° abgekühlt. Es werden portionenweise 440 mg 55-proz. NaH-Dispersin zugefügt und nach Ende der letzten Portion noch 1 Stunde gerührt. Eine Lösung von 1,5 g N-Chlorcarbonylmorpholin in 3 ml N,N-Dimethylformamid wird nun tropfenweise hinzugefügt. Man lässt noch 45 Minuten bei etwa 5° rühren und giesst das Reaktionsgemisch auf 800 ml Eiswasser, wobei das Rohprodukt auskristallisiert. Nach Waschen mit Wasser und Trocknung wird das Rohprodukt durch Chromatographie an Kieselgel und Umkristallisation gereinigt. Man erhält (4,5-Dihydro-7-oxo-8-phenyl-7H-pyrido[1,2-b]thieno[2,3-d]pyridazin-10-yl)methyl-4-morpholincarboxylat vom Smp. 207-210° (Acetonitril).

## Beispiel A

Die Verbindung A, 4-[(10-Chlor-6,7-dihydro-4-oxo-3-phenyl-4H-pyrido[2,1-a]phthalazin-1-yl)carbonyl]-morpholin, kann in an sich bekannter Weise als Wirkstoff zur Herstellung von pharmazeutischen Präparaten nachfolgender Zusammensetzung verwendet werden:

| a) Tabletten | | mg/Tablette |
|---|---|---|
| Verbindung A | | 5 |
| Milchzucker | | 135 |
| Maisstärke | | 51 |
| Polyvinylpyrrolidon | | 8 |
| Magnesiumstearat | | 1 |
| | Tablettengewicht | 200 |

| b) Kapseln | | mg/Kapsel |
|---|---|---|
| Verbindung A | | 10 |
| Milchzucker | | 30 |
| Maisstärke | | 8,5 |
| Talk | | 1 |
| Magnesiumstearat | | 0,5 |
| | Kapselfüllgewicht | 50 |

Anstelle der Verbindung A kann auch die nachfolgend aufgeführte Verbindung als Wirkstoff zur Hertstellung von pharmazeutischen Präparaten obiger Zusammensetzung verwende werden:
Verbindung B: (R)-1-[(10-Chlor-6,7-dihydro-4-oxo-3-phenyl-4H-pyrido[2,1-a]phthalazin-1-yl)carbonyl]-2-(methoxymethyl)pyrrolidin.

## Ansprüche

1. Verbindungen der allgemeinen Formel

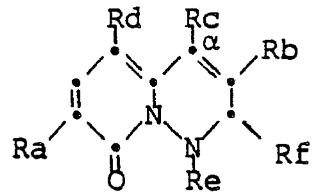

A

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha$-S-CH = CH- (a), $>C_\alpha$-CH = CH-S- (b) oder $>C_\alpha$-CH = CH-CH = CH- (c), Rd die Gruppe der Formel $-(A^1)_m-(CO)_n-(Q^1A^2)_q-R^1$, m, n und q je die Zahl 0 oder 1, $A^1$ niederes Alkylen, $A^2$ niederes Alkylen, eine direkte Bindung oder die Gruppe -CO-, $Q^1$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Hetero- cyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanolakyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N-R^5$ enthalten kann, $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl, Re Wasserstoff, niederes Alkyl, niederes Alkanoyl, Arylcarbonyl, Aryl-niederes Alkanoyl, Aryl-niederes Alkyl oder niederes Alkenyl und Rf Wasserstoff oder niederes Alkyl bedeuten, mit der Massgabe, dass n die Zahl 0 bedeutet, wenn q die Zahl 1 und $A^2$ die Grupe -CO- bedeuten, dass $R^1$ eine von Cyano, Nitro, Halogen oder niederes Alkxoycarbonyl verschiedene Bedeutung hat, wenn q die Zahl 0 und n die Zahl 1 oder wenn q die Zahl 1 und $A^2$ die Gruppe -CO- bedeuten, und dass $R^1$ eine von Hydroxy, Cyano, Nitro, Halogen, niederes Alkoxycarbonyl, niederes Alkoxy und $-NR^3R^4$ verschiedene Bedeutung hat, wenn q die Zahl 1 und $A^2$ eine direkte Bindung bedeuten, und pharmazeutisch annehmbare Säureadditionssalze von Verbindungen der Formel A mit einem oder mehreren basischen Substituenten.

2. Verbindungen nach Anspruch 1, worin Rf Wasserstoff bedeutet.

3. Verbindungen nach Anspruch 1 oder 2, worin Ra Phenyl bedeutet.

4. Verbindungen nach einem der Ansprüche 1-3, worin Rb und Rc zusammen mit dem mit α bezeichneten Kohlenstoffatom eine gegebenenfalls durch Halogen substituierte Gruppe der Formel $>C_\alpha$-S-CH = CH- oder $>C_\alpha$-CH = CH-CH = CH- bedeuten.

5. Verbindungen nach Anspruch 4, worin Rb und Rc zusammen mit dem mit α bezeichneten Kohlenstoffatom die Gruppe der Formel $>C_\alpha$-S-CH = CH- oder $>C_\alpha$-CH = CCl-CH = CH-bedeuten.

6. Verbindungen nach einem der Ansprüche 1-5, worin Rd die Gruppe $-CONR^3R^4$, $R^3$ niederes Alkyl oder niederes Alkoxyalkyl und $R^4$ Wasserstoff oder niederes Alkyl oder $R^3$ und $R^4$ zusamen mit dem Stickstoffatom einen 4-, 5- oder 6-gliedrigen, gegebenenfalls durch eine oder zwei niedere Alkylgruppen oder durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, bedeuten.

7. Verbindungen nach Anspruch 6, worin $R^3$ niederes Alkyl oder niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen oder durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

8. Verbindungen nach Anspruch 7, worin $R^3$ niederes Alkyl oder 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl oder 4-Morpholinyl bedeuten.

9. Verbindungen nach einem der Ansprüche 1-8, worin Re Wasserstoff oder niederes Alkyl bedeutet.

10. 4-[(10-Chlor-6,7-dihydro-4-oxo-3-phenyl-4H-pyrido[2,1-a]phthalazin-1-yl)carbonyl]morpholin.

11. (R)-1-[(10-Chlor-6,7-dihydro-4-oxo-3-phenyl-4H-pyrido[2,1-a]phthalazin-1-yl)carbonyl]-2-(methoxymethyl)pyrrolidin.

12. 1-[(10-Chlor-6,7-dihydro-4-oxo-3-phenyl-4H-pyrido[2,1-a]phthalazin-1-yl)carbonyl]-3-methoxyazetidin.

13. 10-Chlor-6,7-dihydro-N,N-dimethyl-4-oxo-3-phenyl-4H-pyrido[2,1-a]phthalazin-1-carboxamid.

14. (R)-1-[(4,5-Dihydro-7-oxo-8-phenyl-7H-pyrido[1,2-b]thieno[2,3-d]pyridazin-10-yl)carbonyl]-3-methoxypyrrolidin.

15. 1-[(4,5-Dihydro-4-methyl-7-oxo-8-phenyl-7H-pyrido[1,2-b]thieno[2,3-d]pyridazin-10-yl)carbonyl]-3-methoxyazetidin.

16. 1-[(4,5-Dihdyro-5-methyl-7-oxo-8-phenyl-7H-pyrido[1,2-b]thieno[2,3-d]pyridazin-10-yl)carbonyl]-3-methoxyazetidin.

17. 4,5-Dihydro-N,N-dimethyl-7-oxo-8-phenyl-7H-pyrido[1,2-b]thieno[2,3-d]pyridazin-10-carboxamid.

18. Verbindungen nach einem der Ansprüche 1-17 zur Anwendung als therapeutische Wirkstoffe.

19. Verbindungen nach Anspruch 18 zur Anwendung als muskelrelaxierend, sedativ-hypnotisch, anxiolytisch und/oder antikonvulsiv wirksame Stoffe.

20. Verfahren zur Herstellung von Verbindungen nach einem der Ansprüche 1-17 und von pharmazeutisch annehmbaren Säureadditionssalzen davon, dadurch gekennzeichnet, dass man

i) eine Verbindung der allgemeinen Formel

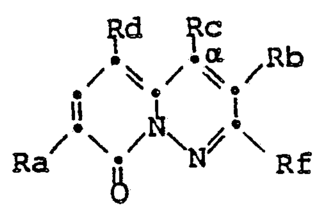

I

worin Ra, Rb, Rc, Rd und Rf die in Anspruch 1 angegebene Bedeutung besitzen,
gegebenenfalls in Gegenwart von Ameisensäure mit einem Alkalimetallborhydrid oder einem Derivat davon reduziert, oder

ii) eine Verbindung der allgemeinen Formel

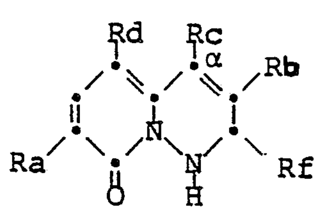

Aa

worin Ra, Rb, Rc, Rd und Rf die in Anspruch 1 angegebene Bedeutung besitzen,
mit einer Verbindung der allgemeinen Formel

Re′-X B

worin X eine Abgangsgruppe und Re′ niederes Alkyl, niederes Alkanoyl, Arylcarbonyl, Aryl-niederes Alkanoyl, Aryl-niederes Alkyl oder niederes Alkenyl bedeuten,
alkyliert bzw. acyliert, oder

iii) eine Verbindung der allgemeinen Formel

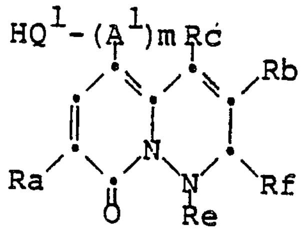

Ab

worin $A^1$, $Q^1$, m, Ra, Rb, Rc, Re und Rf die in Anspruch 1 angegebene Bedeutung besitzen, in Gegenwart einer gegebenenfalls starken Base mit einem reaktiven Derivat einer Carbonsäure der allgemeinen Formel

$R^1$-COOH XIII

worin $R^1$ die in Anspruch 1 angegebene Bedeutung besitzt, umsetzt, und

iv) erwünschtenfalls eine erhaltene Verbindung der Formel A mit einem basischen Substituenten in ein pharmazeutisch annehmbares Säureadditionssalz überführt.

21. Arzneimittel, enthaltend eine Verbindung nach einem der Ansprüche 1-17 und ein therapeutisch inertes Trägermaterial.

22. Muskelrelaxierend, sedativ-hypnotisch, anxiolytisch und/oder antikonvulsiv wirksame Mittel, enthaltend eine Verbindung nach einem der Ansprüche 1-17 und ein therapeutisch inertes Trägermaterial.

23. Verwendung von Verbindung nach einem der Ansprüche 1-17 bei der Bekämpfung oder Verhütung von Krankheiten.

24. Verwendung von Verbindungen nach einem der Ansprüche 1-17 bei der Bekämpfung oder Verhütung von Muskelspannungen, Spannungszuständen, Schlaflosigkeit, Angstzuständen und/oder Konvulsionen.

25. Verwendung von Verbindungen nach einem der Ansprüche 1-17 zur Herstellung von muskelrelaxierend, sedativ-hypnotisch, anxiolytisch und/oder antikonvulsiv wirksamen Mitteln.

Patentansprüche für folgende Vertragsstaaten; GR, ES

1. Verfahren zur Herstellung von Verbindungen der allgemeinen Formel

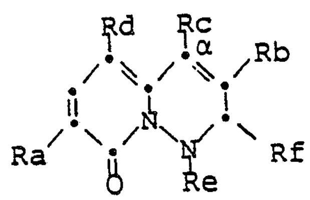

A

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit $\alpha$ bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha$-S-CH=CH- (a), $>C_\alpha$-CH=CH-S- (b) oder $>C_\alpha$-CH=CH-CH=CH- (c), Rd die Gruppe der Formel $-(A^1)_m-(CO)_n-(Q^1A^2)_q-R^1$, m, n und q je die Zahl 0 oder 1, $A^1$ niederes Alkylen, $A^2$ niederes Alkylen, eine direkte Bindung oder die Gruppe -CO-, $Q^1$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo,

Carbamoyl, mono- oder di(niederes Alkyl)carbamoyl oder durch niederes Alkylendioxy substituierte ($C_{3-7}$)-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe >N-$R^5$ enthalten kann, $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl, Re Wasserstoff, niederes Alkyl, niederes Alkanoyl, Arylcarbonyl, Aryl-niederes Alkanoyl, Aryl-niederes Alkyl oder niederes Alkenyl und Rf Wasserstoff oder niederes Alkyl bedeuten, mit der Massgabe, dass n die *Zahl 0 bedeutet*, wenn q die *Zahl* 1 und $A^2$ die Gruppe -CO- bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe -CO- bedeuten, und dass $R^1$ eine von Hydroxy, Cyano, Nitro, Halogen, niederes Alkoxycarbonyl, niederes Alkoxy und -$NR^3R^4$ verschiedene Bedeutung hat, wenn q die Zahl 1 und $A^2$ eine direkte Bindung bedeuten, und von pharmazeutisch annehmbaren Säureadditionssalzen von Verbindungen der Formel A mit einem oder mehreren basischen Substituenten, dadurch gekennzeichnet, dass man

i) eine Verbindung der allgemeinen Formel

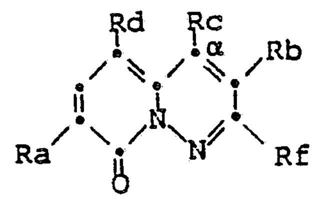

I

worin Ra, Rb, Rc, Rd und Rf obige Bedeutung besitzen,
gegebenenfalls in Gegenwart von Ameisensäure mit einem Alkalimetallborhydrid oder einem Derivat davon reduziert, oder

ii) eine Verbindung der allgemeinen Formel

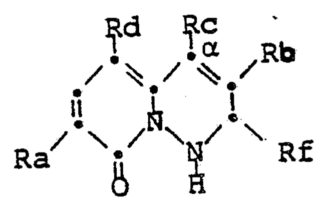

Aa

worin Ra, Rb, Rc, Rd und Rf obige Bedeutung besitzen,
mit einer Verbindung der allgemeinen Formel

Re′-X B

worin X eine Abgangsgruppe und Re′ niederes Alkyl, niederes Alkanoyl, Arylcarbonyl, Aryl-niederes Alkanoyl, Aryl-niederes Alkyl oder niederes Alkenyl bedeuten,
alkyliert bzw. acyliert, oder

iii) eine Verbindung der allgemeinen Formel

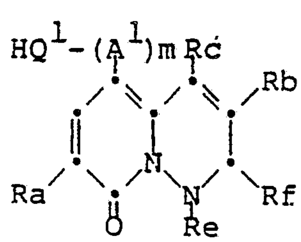

Ab

worin $A^1$, $Q^1$, m, Ra, Rb, Rc, Re und Rf obige Bedeutung besitzen, in Gegenwart einer gegebenenfalls starken Base mit einem reaktiven Derivat einer Carbonsäure der allgemeinen Formel

$R^1$-COOH XIII

worin $R^1$ obige Bedeutung besitzt, umsetzt, und

iv) erwünschtenfalls eine erhaltene Verbindung der Formel A mit einem basischen Substituenten in ein pharmazeutisch annehmbares Säureadditionssalz überführt.

2. Verfahren nach Anspruch 1, worin Rf Wasserstoff bedeutet.

3. Verfahren nach Anspruch 1 oder 2, worin Ra Phenyl bedeutet.

4. Verfahren nach einem der Ansprüche 1-3, worin Rb und Rc zusammen mit dem mit α bezeichneten Kohlenstoffatom eine gegebenenfalls durch Halogen substituierte Gruppe der Formel >$C_\alpha$-S-CH=CH- oder >$C_\alpha$-CH=CH-CH=CH- bedeuten.

5. Verfahren nach Anspruch 4, worin Rb und Rc zusammen mit dem mit α bezeichneten Kohlenstoffatom die Gruppe der Formel >$C_\alpha$-S-CH=CH- oder >$C_\alpha$-CH=CCl-CH=CH- bedeuten.

6. Verfahren nach einem der Ansprüche 1-5, worin Rd die Gruppe -CON$R^3R^4$, $R^3$ niederes Alkyl oder niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen oder durch eine Hydroxy-, niedere Alkoxy-, niedere Hydroxyalkyl- oder niedere Alkoxyalkylgruppe substituierte, gesättigten N-Heterocyclus, der als Ringglied noch ein Sauerstoffatom enthalten kann, bedeuten.

7. Verfahren nach Anspruch 6, worin $R^3$ niederes Alkyl oder niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen oder durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

8. Verfahren nach Anspruch 7, worin $R^3$ niederes Alkyl oder 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl,m 3-(niederes Alkoxy)-1-pyrrolidinyl, 2-(niederes Alkoxyalkyl)-1-pyrrolidinyl oder 4-Morpholinyl bedeuten.

9. Verfahren nach einem der Ansprüche 1-8, worin Re Wasserstoff oder niederes Alkyl bedeutet.

10. Verfahren nach Anspruch 1, dadurch gekennzeichnet, dass man 4-[(10-Chlor-6,7-dihydro-4-oxo-3-phenyl-4H-pyrido[2,1-a]phthalazin-1-yl)carbonyl]morpholin herstellt.

11. Verfahren nach Anspruch 1, dadurch gekennzeichnet, dass man (R)-1-[(10-Chlor-6,7-dihydro-4-oxo-3-phenyl-4H-pyrido[2,1-a]phthalazin-1-yl)carbonyl]-2-(methoxymethyl)pyrrolidin herstellt.

12. Verfahren nach Anspruch 1, dadurch gekennzeichnet, dass man 1-[(10-Chlor-6,7-dihydro-4-oxo-3-phenyl-4H-pyrido[2,1-a]phthalazin-1-yl)carbonyl]-3-methoxyazetidin herstellt.

13. Verfahren nach Anspruch 1, dadurch gekennzeichnet, dass man 10-Chlor-6,7-dihydro-N,N-dimethyl-4-oxo-3-phenyl-4H-pyrido[2,1-a]phthalazin-1-carboxamid herstellt.

14. Verfahren nach Anspruch 1, dadurch gekennzeichnet, dass man (R)-1-[(4,5-Dihydro-7-oxo-8-phenyl-7H-pyrido[1,2-b]thieno[2,3-d]pyridazin-10-yl)carbonyl]-3-methoxypyrrolidin herstellt.

15. Verfahren nach Anspruch 1, dadurch gekennzeichnet, dass man 1-[4,5-Dihydro-4-methyl-7-oxo-8-phenyl-7H-pyrido[1,2-b]thieno[2,3-d]pyridazin-10-yl)carbonyl]-3-methoxyazetidin herstellt.

16. Verfahren nach Anspruch 1, dadurch gekennzeichnet, dass man 1-[4,5-Dihydro-5-methyl-7-oxo-8-phenyl-7H-pyrido[1,2-b]thieno[2,3-d]pyridazin-10-yl)carbonyl]-3-methoxyazetidin herstellt.

17. Verfahren nach Anspruch 1, dadurch gekennzeichnet, dass man 4,5-Dihydro-N,N-dimethyl-7-oxo-8-phenyl-7H-pyrido[1,2-b]thieno[2,3-d]pyridazin-10-carboxamid herstellt.

18. Verfahren zur Herstellung von Arzneimitteln, insbesondere von muskelrelaxierend, sedativ-hypnotisch, anxiolytisch und/oder antikonvulsiv wirksamen Mitteln, dadurch gekennzeichnet, dass man eine Verbindung der in Anspruch 1 definierten Formel A oder ein pharmazeutisch annehmbares Säureadditionssalz einer Verbindung der Formel A mit einem oder mehreren basischen Substituenten und gegebenenfalls einen anderen therapeutisch wirksamen Stoff zusammen mit einem therapeutisch inerten Trägermaterial in eine galenische Darreichungsform bringt.

19. Verwendung von Verbindungen der in Anspruch 1 definierten Formel A oder von pharmazeutisch annehmbaren Säureadditionssalzen von Verbindungen der Formel A mit einem oder mehreren basischen Substituenten zur Herstellung von muskelrelaxierend, sedativ-hypnotisch, anxiolytisch und/oder antikonvulsiv wirksamen Mitteln.